# EUROPEAN PATENT SPECIFICATION

(11) **EP 1 342 719 B1**
(45) Date of publication and mention of the grant of the patent: **13.04.2011**
(21) Application number: 03010776.7
(22) Date of filing: 26.02.1993
(51) Int. Cl.: C07D 243/24, A61K 31/5513, A61P 35/00

(54) **Benzodiazepine derivatives useful as CCK-Receptor Antagonists**
Benzodiazepin-Derivate, verwendbar als CCK-Rezeptor Antagonisten
Dérivés de benzodiazépine utiles comme antagonistes de récepteurs-CCK

(30) Priority: 27.02.1992 GB 9204221; 16.06.1992 GB 9212740
(43) Date of publication of application: 10.09.2003
(62) Divisional of application: 93905480.5
(73) Proprietor: Ferring BV, 2132 JX Hoofddorp (NL)
(72) Inventor: Ryder, Hamish, Bitterne, Southampton SO2.5AQ (GB); Semple, Graeme, Marchwood, Southampton SO4.4XU (GB); Kendrick, David Alan, Eastleigh, Hampshire SO5.5QU (GB); Szelke, Michael, Braishfield, Romsey (GB); Satoh, Masato, Tsukuba-shi, Ibaraki 305 (JP); Ohta, Mitsuaki, Tsukuba-gun, Ibaraki 300-24 (JP); Miyata, Keiji, Tsukuba-shi, Ibaraki 305 (JP); Nishida, Akito, Tsukuba-shi, Ibaraki 305 (JP)
(74) Representative: Bates, Philip Ian

(56) References cited:
- EP-A- 0 434 360
- EP-A- 0 434 369
- EP-A- 0 445 976
- EP-A- 0 508 797
- EP-A- 0 508 799

## Description

This invention relates to benzodiazepine derivatives which are useful as drugs exhibiting antagonism at the gastrin and/or CCK-B receptor and to their production.

Many benzodiazepine derivatives have been described in the course of development of psychotropic drugs which act as agonists at the "benzodiazepine receptor" in the central nervous system. More recently benzodiazepine derivatives have been described which act as antagonists at the CCK-A (cholocystokinin-A) and CCK-B receptors. It was further reported that those compounds which were selective antagonists for the CCK-B receptor were able to reduce the secretion of gastric acid in response to the administration of pentagastrin (V.J. Lotti & R.S.L. Chang, Eur.J.pharmacol. 162, 273-280. 1989). Examples of benzodiazepine derivatives which act as antagonists at the CCK-B receptor are disclosed in for example. U.S.Patent No.4.820.834. EP0445976, EP0434360, EP0434369 and EP0508797 disclose benzodiazepine analogs.

The compounds of the present invention are novel. They differ from the compounds described in U.S.Patent No.4.820.834. particularly in the nature of the substituent at position 1 of the benzodiazepine nucleus. The present invention includes compounds of better pharmacological characteristics than those described in U.S.Patent No.4.820.834; preferred compounds of the invention have a higher affinity for the CCK-B receptor and/or discriminate more selectively between the CCK-B and CCK-A receptors than the previously described compounds.

The present invention provides a benzodiazepine derivate of formula I, or a pharmaceutically acceptable salt thereof : 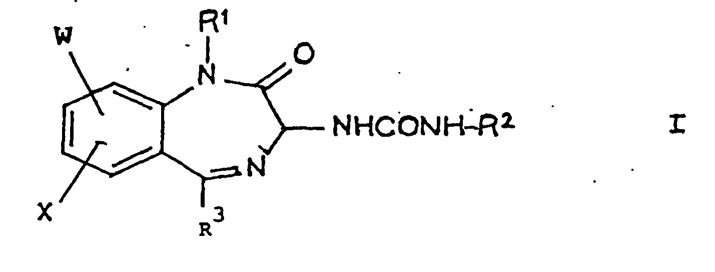 wherein
(a) R¹ is -CH₂CHOH(CH₂)ₐR⁴ or a ketone group -CH₂CO(CH₂)ₐR⁵ in which a is 0 or 1 and R⁴ and R⁵ are selected from alkyl groups of up to 8 carbon atoms;
(b) R² and R³ are independently selected from optionally substituted phenyl and pyridyl, the optional substituents being selected from halogen atoms and hydroxy, amino, nitro, carboxylic acid, carboxamido and cyano groups and alkyl, alkoxy, alkylamino and dialkylamino groups in which the or each alkyl group is of up to 8 carbon atoms; and
(c) W and X are selected independently from halogen and hydrogen atoms and C₁-C₈ alkyl and C₁-C₈ alkoxy groups.

Herein all "alkyl", "cycloalkyl", "acyl" and "alkoxy" groups are of up to 8 carbon atoms, and "halogen" may for example be fluorine, chlorine, bromine or iodine. Preferably at least one of R² and R³ is unsubstituted, monosubstituted or disubstituted phenyl or unsubstituted, monosubstituted or disubstituted 2-, 3-, or 4-pyridyl. Preferably one (most preferably each) of W and X is a hydrogen atom.

R⁴ is alkyl (e.g. C₄ - C₇, linear or branched). R⁵ is alkyl (e.g. C₁ - C₃).

Examples of alkyl groups include tert-butyl.

Examples of substitutents on the aromatic residues (R², R³) include halogen atoms (e.g. fluorine, chlorine, etc); hydroxy, amino, nitro, carboxylic acid and cyano groups; and alkyl, alkoxy, alkylamino and dialkylamino groups in which the or each alkyl component is preferably of up to 6 (e.g. up to 3) carbon atoms (methyl, ethyl etc); for substituted R², meta-substitution is preferred.

Most preferably R² is unsubstituted phenyl; phenyl having a meta substituent chosen from F, Cl, Br, OH, OCH₃, NH₂, NMe₂, NO₂, Me, -(CH₂)_{c}-CO₂H, CN, NHMe, NMeEt, NEt₂, CH₂NMe₂, NHCHO and - (CH₂)_{c}SO₃H where c is 0-2; or 2-, 3- or 4-pyridyl optionally with a substituent selected from F, Cl, CH₃ and CO₂H.

Most preferably R³ is phenyl or 2-, 3- or 4-pyridyl.

W and X are preferably both H, but when either is alkyl or alkoxy it is preferably of 1 to 3 carbon atoms.

The compounds of this invention all have at least one stereogenic centre and so can exist as optical isomers. It should be understood that these isomers, either separately or as mixtures, are included within the scope of this invention. In addition, the compounds of this invention can form salts with inorganic or organic acids or, in some cases, bases. Examples of such salts would include chlorides, sulphates and acetates, or sodium and potassium salts. These salts should also be understood to be included within the scope of this invention. In preferred compounds according to the invention, the absolute configuration at the 3-position of the benzodiazepine is R (as shown in IV).

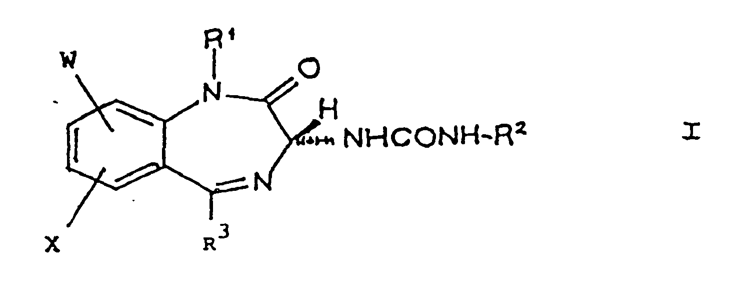

Compounds according the invention act as CCK-B and gastrin receptor antagonists. They may be used as drugs for the treatment of diseases induced by the failure of a physiological function controlled by gastrin, such as gastric and duodenal ulcers, gastritis, reflux esophagitis, gastric and colon cancers, and Zollinger-Ellison syndrome; there may be no side effects arising from CCK-A receptor interaction. They may be used as drugs for the treatment of diseases induced by the failure of physiological function controlled by the central CCK-B receptor (e.g. for the reduction of anxiety or for appetite regulation).

Amongst preferred compounds according to the invention are those listed below and salts thereof. Some of the compounds are exemplified hereinafter as indicated against the individual compounds concerned.
1. N-((3RS)-1-*tert*-Butylcarbonylmethyl-2,3-dihydro-2-oxo-5-phenyl-1H-1,4-benzodiazepin-3-yl)-N'-(3-methylphenyl) urea (Example 2);
2. N-((3RS)-1-Diethylmethylcarbonylmethyl-2,3-dihydro-2-oxo-5-phenyl-1H-1,4-benzodiazepin-3-yl)-N'-(3-methylphenyl) urea (Example 3);
23. N-((3RS)-1-*tert*-Butylcarbonylmethyl-2,3-dihydro-2-oxo-5-phenyl-1H-1,4-benzodiazepin-3-yl)-N'-(3-cyanophenyl) urea (Example 50);
38. N-((3R)-1-*tert*-Butylcarbonylmethyl-2,3-dihydro-2-oxo-5-phenyl-1H-1,4-benzodiazepin-3-yl)-N'-(3-methylphenyl) urea (Example 26);
39. N-((3RS)-1-*tert*-Butylcarbonylmethyl-2,3-dihydro-2-oxo-5-(2-pyridyl)-1H-1,4-benzodiazepin-3-yl)-N'-(3-methylphenyl) urea (Example 27);
40. N-((3RS)-1-*tert*-Butylcarbonylmethyl-2,3-dihydro-2-oxo-5-phenyl-1H-1,4-benzodiazepin-3-yl)-N'-(3-carboxyphenyl) urea (Example 28);
41. N-((3RS)-1-*tert*-Amylcarbonylmethyl-2,3-dihydro-2-oxo-5-phenyl-1H-1,4-benzodiazepin-3-yl)-N'-(3-methylphenyl) urea (Example 36);
42. N-((3RS)-1-*tert*-Amylcarbonylmethyl-2,3-dihydro-2-oxo-5-phenyl-1H-1,4-benzodiazepin-3-yl)-N'-(3-carboxyphenyl) urea (Example 37);
43. N-((3RS)-1-*tert*-Butylcarbonylmethyl-2,3-dihydro-2-oxo-5-(2-pyridyl)-1H-1,4-benzodiazepin-3-yl)-N'-(3-dimethylaminophenyl) urea (Example 38);
44. N-((3RS)-1-*tert-*Butylcarbonylmethyl-2,3-dihydro-2-oxo-5-(2-pyridyl)-1H-1,4-benzodiazepin-3-yl)-N'-(3-carboxyphenyl) urea (Example 39);
46. N-((3RS)-1-*tert*-Butylcarbonylmethyl-2,3-dihydro-2-oxo-5-phenyl-1H-1,4-benzodiazepin-3-yl)-N'-(3-dimethylaminophenyl) urea (Example 41);
48. N-((3RS)-1-*tert*-Butylcarbonylmethyl-2,3-dihydro-2-oxo-5-phenyl-1H-1,4-benzodiazepin-3-yl)-N'-(3-methoxyphenyl) urea (Example 43);
50. N-((3RS)-1-*tert*-Butylcarbonylmethyl-2,3-dihydro-2-oxo-5-phenyl-1H-1,4-benzodiazepin-3-yl)-N'-(3-nitrophenyl) urea (Example 45);
51. N-((3RS)-1-*tert*-Butylcarbonylmethyl-2,3-dihydro-2-oxo-5-phenyl-1H-1,4-benzodiazepin-3-yl)-N'-(3-formylaminophenyl) urea (Example 47);
52. N-((3R)-1-((2R)-2-Hydroxy-3,3-dimethylbutyl)-2,3-dihydro-2-oxo-5-phenyl-1H-1,4-benzodiazepin-3-yl)-N'-(3-methylphenyl) urea (Example 48);
53. N-((3R)-1-((2S)-2-Hydroxy-3,3-dimethylbutyl)-2,3-dihydro-2-oxo-5-phenyl-1H-1,4-benzodiazepin-3-yl)-N'-(3-methylphenyl)urea;
55. N-((3RS)-1-*tert*-Butylcarbonylmethyl-2,3-dihydro-2-oxo-5-(2-chlorophenyl)-1H-1,4-benzodiazepin-3-yl)-N'-(3-methylphenyl) urea (Example 54);
56. N-((3RS)-1-Isopropylcarbonylmethyl-2,3-dihydro-2-oxo-5-phenyl-1H-1,4-benzodiazepin-3-yl)-N'-(3-methylphenyl) urea (Example 55);
57. N-((3R)-1-*tert*-Butylcarbonylmethyl-2,3-dihydro-2-oxo-5-phenyl-1H-1,4-benzodiazepin-3-yl)-N'-(3-methylaminophenyl) urea (Example 56);
58. N-((3RS)-1-*tert*-Butylcarbonylmethyl-2,3-dihydro-2-oxo-5-(2-pyridyl)-1H-1,4-benzodiazepin-3-yl)-N'-(3-methylaminophenyl) urea (Example 57);
59. N-((3R)-1-*tert*-Butylcarbonylmethyl-2,3-dihydro-2-oxo-5-phenyl-1H-1,4-benzodiazepin-3-yl)-N'-(3-(N-ethyl-N-methylamino)phenyl) urea (Example 58);
60. N-((3R)-1-*tert*-Butylcarbonylmethyl-2,3-dihydro-2-oxo-5-phenyl-1H-1,4-benzodiazepin-3-yl)-N'-(3-diethylaminophenyl) urea (Example 59);
61. N-((3RS)-1-*tert*-Butylcarbonylmethyl-2,3-dihydro-2-oxo-5-phenyl-1H-1,4-benzodiazepin-3-yl)-N'-(3-dimethylaminomethylphenyl) urea (Example 60);
62. N-((3RS)-1-*tert*-Butylcarbonylmethyl-2,3-dihydro-2-oxo-5-(2-pyridyl)-1H-1,4-benzodiazepin-3-yl)-N'-(3-(N-ethyl-N-methylamino)phenyl) urea (Example 61);
63. N-((3RS)-1-*tert*-Butylcarbonylmethyl-2,3-dihydro-2-oxo-5-(2-pyridyl)-1H-1,4-benzodiazepin-3-yl)-N'-(3-dimethylaminomethylphenyl) urea (Example 62);
64. N-((3RS)-1-*tert*-Butylcarbonylmethyl-2,3-dihydro-2-oxo-5-(2-pyridyl)-1H-1,4-benzodiazepin-3-yl-N'-(3-diethylaminophenyl urea (Example 63);
65. N-((3R)-1-*tert*-Butylcarbonylmethyl-2,3-dihydro-2-oxo-5-phenyl-1H-1,4-benzodiazepin-3-yl)-N'-(3-dimethylaminophenyl) urea (Example 64);
66. N-((3RS)-1-*tert*-Butylcarbonylmethyl-2,3-dihydro-2-oxo-5-(4-methylphenyl)-1H-1,4-benzodiazepin-3-yl)-N'-(3-methylphenyl) urea (Example 65);
67. N-((3R)-1-*tert*-Butylcarbonylmethyl-2,3-dihydro-2-oxo-5-phenyl-1H-1,4-benzodiazepin-3-yl)-N'-(3-aminophenyl) urea (Example 66);
68. N-((3R)-1-*tert*-Butylcarbonylmethyl-2,3-dihydro-2-oxo-5-(2-pyridyl)-1H-1,4-benzodazepin-3-yl)-N'-(3-methylphenyl) urea;
69. N-((3R)-1-*tert*-Butylcarbonylmethyl-2,3-dihydro-2-oxo-5-(2-pyridyl)-1H-1,4-benzodiazepin-3-yl)-N'-(3-aminophenyl) urea;
70. N-((3R)-1-*tert*-Butylcarbonylmethyl-2,3-dihydro-2-oxo-5-(2-pyridyl)-1H-1,4-benzodiazepin-3-yl)-N'-(3-methylaminophenyl) urea;
71. N-((3R)-1-*tert*-Butylcarbonylmethyl-2,3-dihydro-2-oxo-5-(2-pyridyl)-1H-1,4-benzodiazepin-3-yl)-N'-(3-dimethylaminophenyl) urea;
72. N-((3RS)-1-*tert*-Butylcarbonylmethyl-2,3-dihydro-2-oxo-5-phenyl-1H-1,4-benzodiazepin-3-yl)-N'-(3-aminophenyl) urea;
73. N-((3RS)-1-*tert*-Butylcarbonylmethyl-2,3-dihydro-2-oxo-5-phenyl-1H-1,4-benzodiazepin-3-yl)-N'-(3-methylaminophenyl) urea;
74. N-((3RS)-1-*tert*-Butylcarbonylmethyl-7-chloro-2,3-dihydro-2-oxo-5-phenyl-1H-1,4-benzodiazepin-3-yl)-N'-(3-methylphenyl) urea (Example 67);
75. N-((3RS)-1-*tert*-Butylcarbonylmethyl-7-chloro-2,3-dihydro-2-oxo-5-phenyl-1H-1,4-benzodiazepin-3-yl)-N'-(3-dimethylaminophenyl) urea (Example 68);
76. N-((3RS)-1-*tert*-Butylcarbonylmethyl)-7-chloro-2,3-dihydro-2-oxo-5-(2-chlorophenyl)-1H-1,4-benzodiazepin-3-yl)-N'-(3-methylphenyl) urea (Example 69);
77. N-((3RS)-1-*tert*-Butylcarbonylmethyl-2,3-dihydro-8-methyl-2-oxo-5-phenyl-1H-1,4-benzodiazepin-3-yl)-N'-(3-methylphenyl) urea (Example 70);
78. N-((3RS)-1-*tert*-Butylcarbonylmethyl-2,3-dihydro-2-oxo-5-phenyl-1H-1,4-benzodiazepin-3-yl)-N'-(3-(N-ethyl-N-methylaminophenyl) urea;
79. N-((3RS)-1-*tert*-Butylcarbonylmethyl-2,3-dihydro-2-oxo-5-phenyl-1H-1,4-benzodiazepin-3-yl)-N'-(3-diethylaminophenyl) urea.

The compounds of the invention can be prepared according to the route outlined in Scheme I below. 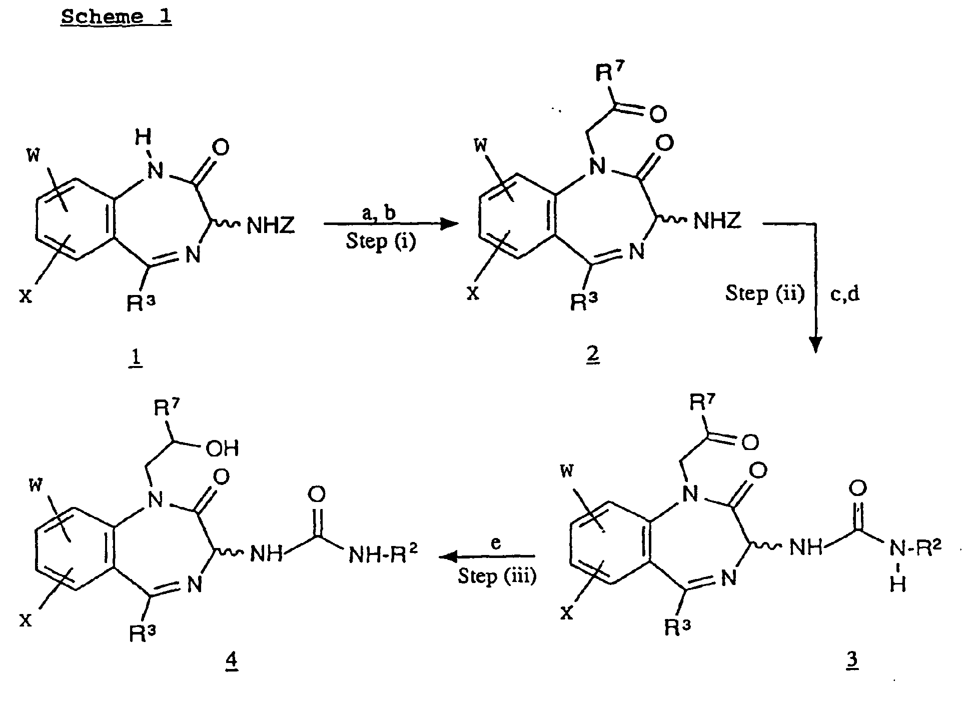
- Reagents:: (a)NaH,DMF; (b) R₇COCH₂Br; (c) H₂, Pd-C or HBr; (d) R²NCO; (e) NaBH₄
(R⁷ represents R⁴(CH₂)ₐ or R⁵(CH₂)ₐ)

When R³ = Ph, the starting material 1 is a known compound [M.G. Bock et al. J. Org. Chem. 52, 3232-3239, 1987]. For other examples of R³, such as pyridyl, the starting material 1 can be prepared in a manner analogous to that described by Bock *et al*. For simplicity, in the specific examples which follow, the compound 1 (R³ = Ph) is referred to as the Bock benzodiazepine.

In step (i) the compound 1 is deprotonated with a strong base (typically sodium hydride) and then reacted with a bromomethyl ketone R⁷COCH₂Br. In general, these ketones are not commercially available, but can be prepared from commercially available carboxylic acids or carboxylic acid chlorides by the route outlined in Scheme 2. 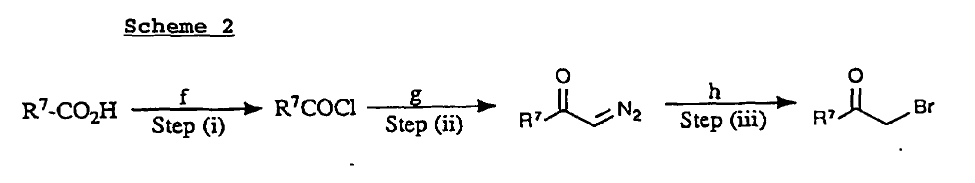 Reagents: (f) SOCl₂; (g) CH₂N₂; (h) HBr

The alkylated benzodiazepines 2 (Scheme 1) are then deprotected. Scheme 1 illustrates the case when the amino-group is protected by a Z group (Z = benzyloxycarbonyl) which can be removed by catalytic hydrogenolysis or acidic hydrolysis.

When a protecting group other than Z is used the deprotection step may need to be changed appropriately. The deprotected benzodiazepine is then treated with an aryl isocyanate (R²NCO). When R² = 3-methylphenyl for example, this leads directly to compounds 3 which are listed above (e.g. compounds 1 - 10, etc). When R² contains a protected functional group (e.g. a carboxylic acid protected as an ester) this group must be unmasked to give the listed compound. The ketone 3 can be reduced by, for example, sodium borohydride to give the corresponding alcohol 4 (e.g. compounds 18 - 21, etc).

In some cases it is preferable to prepare the urea by an altenate route. This is outlined in Scheme 3. 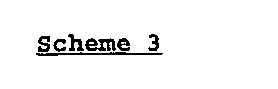 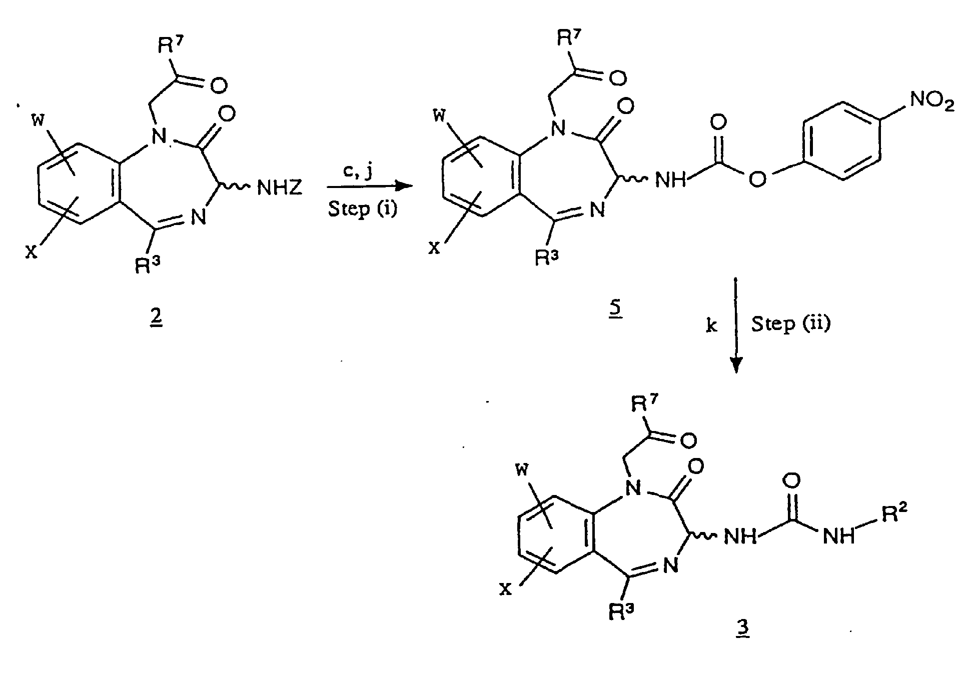 Reagents; (j) p-O₂NC₆H₄OCOCl; (k) R²NH₂

The protected benzodiazepine derivative 2 is protected as before {Scheme 1, step (ii)} but is then treated with p-nitrophenyl chloroformate to give the p-nitrophenyl carbamate 5. In step (ii) this is reacted with an amine R²NH₂ to give the urea 3. This route is particularly useful when the isocyanate R²NCO is not commercially available but the amine R²NH₂ is.

These general methods will now be further illustrated with specific, non-limiting examples. Reference Examples are included but are not embodiments of the present invention.

### REFERENCE EXAMPLE 1

### N-((3RS)-1-Cyclobutylcarbonylmethyl-2,3-dihydro-2-oxo-5-phenyl-1H-1,4-benzodiazepin-3-yl)-N'(3-methylphenyl) urea. Compound 3

### 1A

### Bromomethyl cyclobutyl ketone (Scheme 2, Steps (i) - (iii))

To an ice-cold solution of cyclobutanecarboxylic acid (1.5 g, 15 mmol) in Et₂O (10 ml) was added N,N-diisopropylethylamine (2.9 ml, 16 mmol) followed by thionyl chloride, (1.24 ml, 17 mmol). The mixture was stirred at O°C for 45 min. then poured into an ice-cold solution of CH₂N₂ (prepared from Diazald^{®}, 14.1 g, 66 mmol) in Et₂O, and the resulting mixture was allowed to warm to room temperature over 2 hrs. A saturated solution of HBr in Et₂O was added dropwise until no more nitrogen was evolved. The resulting ethereal solution of bromomethyl ketone was washed successively with satd. KHCO₃, water and brine, filtered (Whatman^{®} 1 PS phase separator), and concentrated in vacuo. The crude product was purified by bulb-to-bulb distillation (100°C, 5 mm Hg), to give the title ketone as a colourless, mobile oil (1.13 g, 44%).
- NMR: (CDCl₃) δ 3.86 (2H, s); 3.57 (1H, quintet, J = 8.5 Hz); 2.5 - 2.2 (6H, m) ; 2 .2-1. 8 (2H, m).

### 1B

### (3RS)-3-Benzyloxycarbonylamino-1-cyclobutylcarbonyl-methyl-2,3-dihydro-5-phenyl-1H-1,4-benzodiazepin-2-one {Scheme 1, step (i)}

To a stirred solution of (3RS)-3-benzyloxycarbonyl-amino-2,3-dihydro-5-phenyl-1H-1,4-benzodiazepin-2-one [M.G. Bock et al. J. Org. Chem. 52, 3232-3239, 1987] (578 mg, from 605 mg of monohydrate azeotroped with DMF x 3, 1.5 mmol) in DMF (5ml), cooled to -10°C under N₂, was added sodium hydride (63 mg of 80% dispersion in oil, 2.1 mmol). The mixture was stirred at -10°C for 30 min, when a solution of the bromomethyl ketone of Example 1A (398 mg, 2.25 mmol) in DMF (2 ml) was added. The resulting mixture was allowed to warm to room temperature with stirring over 1 hr, then poured into dilute aq. HCl (100 ml). The mixture was extracted once with EtOAc, and the organic layer was washed with water and brine, filtered (Whatman^{®} 1 PS phase separator), and concentrated *in vacuo.* The crude product was purified by flash chromatography on silica gel (eluant EtOAc: 60-80 pet. ether 40:60 v/v) to give the title benzodiazepine as a glassy solid (490 mg, 68%).
- R_{f}: (EtOAc: 60-80 pet. ether 50:50) 0.54.
- NMR: (CDCl₃) δ 7.8 - 7.2 (14H, m); 6.70 (1H, d, J = 8 Hz); 5.47 (1H, d, J = 8 Hz); 5.18 (2H, s); 4.69 (H, d, J = 7.5 Hz); 4.60 (1H, d, J = 7.5 Hz);3.36 (1H, quintet, J = 8 Hz); 2.5 - 1.8 (6H, m).

### 1C

### N-((3RS)-1-Cyclobutylcarbonylmethyl-2 3-dihydro-2-oxo-5-phenyl-1H-1,4-benzodiazepin-3-yl)-N'-(3-methyl-phenyl) urea (Scheme 1, step (ii))

To a deoxygenated solution of the benzodiazepine of Example 1B (490 mg, 1.02 mmol) in glacial acetic acid (25 ml) was added 5% palladium-on-carbon catalyst (ca. 200 mg). Hydrogen gas was bubbled through the suspension for 4 hrs, and the reaction was terminated by nitrogen degassing. The mixture was filtered, and the catalyst residue was washed with methanol. The combined filtrates were evaporated in vacuo, and traces of solvent were finally removed by coevaporation with toluene. The residue was taken up in CH₂Cl₂ (25 ml) and stirred at room temperature. To this solution was added m-tolyl isocyanate (0.13 ml, 1.02 mmol), and stirring was continued for 2 hrs. The solvent was evaporated *in vacuo,* and the crude product was purified by flash chromatography on silica gel (eluant EtOAc: 60-80 pet. ether 40:60 v/v). Finally the product was taken up in acetic acid and lyophilised to give the title benzodiazepine as a white powder (203 mg, 41%, >95% pure by HPLC).
- R_{f}: (EtOAc: 60-80 pet. ether 35:65) 0.12.
- NMR: (CDCl₃) δ 7.82 (2H, d, J = 7 Hz); 7.8 - 7.2 (11H, m); 7.09 (1H, d, J = 8 Hz); 6.99 (1H, d, J = 7 Hz); 5.83 (1H, d, J = 8 Hz); 4.78 (2H, s); 3.44 (1H, quintet, J = 8.5 Hz); 2.43 (3H, s); 2.4 - 1.9 (6H, m).
- M.S.: (FAB, +ve ion) m/e 481.8 (M+H).

### EXAMPLE 2

### N-((3RS)-1-tert-Butylcarbonylmethyl-2,3-hydro-2-oxo-5-phenyl-1H-1,4-benzodiaze-pin-3-yl)-N'-(3-methyliphenyl) urea. Compound 1

### 2A

### tert-Butyl bromomethyl ketone (Scheme 2, steps (ii) - (iii)

To an ice-cold solution of CH₂N₂ (prepared from Diazald^{®}, 10.4 g, 49 mmol) in Et₂O was added pivaloyl chloride (2 ml, 16 mmol). The solution was allowed to warm to room temperature with stirring over 3 hrs, when a saturated solution of HBr in EtOAc was added until no more nitrogen was evolved. The solution was washed with brine, filtered (Whatman^{®} 1 PS phase separator), and concentrated in vacuo to give the title ketone as a mobile, pale brown oil (3.47 g, 82% pure by NMR, remainder EtOAc, 99%).
- NMR: (CDCl₃) δ 4.15 (2H, s); 1.20 (9H, s).

### 2B

### (3RS)-3-Benzyloxycarbonylamino-1-tert-butylcarbonylmethyl-2,3-dihydro-5-phenyl-1H-1,4-benzodiazepin-2-one, (Scheme 1, step (i)

This was prepared following the method of Reference Example 1B using the Bock benzodiazepine (578 mg, 1.5 mmol), sodium hydride (63 mg of 80% dispersion in oil, 2.1 mmol) and the bromomethyl ketone of Example 2A (491 mg, 82% pure, 2.25 mmol). The crude product was purified by flash chromatography on silica gel (eluant EtOAc: 60-80 pet. ether 50:50 v/v) to give the title benzodiazepine as a glassy solid (700 mg, 97%).
- R_{f}: (EtOAc: 60-80 pet. ether 50:50) 0.56.
- NMR: (CDCl₃) δ 7.8 - 7.2 (14H, m) ; 6.74 (1H, d, J = 8 Hz); 5.53 (1H, d, J = 8 Hz); 5.23 (2H, s); 5.05 (1H, d, J = 18 Hz; 4.77 (1H, d, J = 18 Hz); 1.33 (9H, s).

### 2C

### N-(3RS)-1-tert-Butylcarbonylmethyl-2,3,-dihydro-2-oxo-5-phenyl-1H-1,4-benzodiazenin-3-yl)-N'-(3-methylphenyl) urea (Scheme 1, step (ii))

This was prepared following the method of Reference Example 1C using the benzodiazepine of Example 2B (700 mg, 1.45 mmol), 5% palladium-on-carbon (ca. 300 mg), and m-tolyl isocyanate (0.19 ml, 1.5 mmol). The crude product was purified by flash chromatography on silica gel (eluant EtOAc: 60-80 pet. ether 40:60 v/v), taken up in acetic acid, and lyophilised to give the title benzodiazepine as a white powder (233 mg, 33%, >97% pure by HPLC).
- R_{f}: (EtOAc: 60- 80 pet. ether 40:60) 0.31.
- NMR: (CDCl₃) δ 7.72 (2H, d, J = 8 Hz); 7.7 - 7.0 (12H, m); 6.87 (1H, d, J = 7 Hz); 5.73 (1H, d, J = 8 Hz); 4.92 (1H, d, J = 18 Hz); 4.82 (1H, d, J = 18 Hz) ; 2.32 (3H, s); 1.26 (9H, s).
- M.S.: (FAB, +ve ion) m/e 483.2 (M+H).

### EXAMPLE 3

### N-((3RS)-1-Diethylmethylcarbonylmethyl-2,3-dihydro-2-oxo-5-phenyl-1H-1,4-benzodiazepin-3-yl)-N'(3-methylphenyl) urea. Compound 2

### 3A

### Bromomethyl diethylmethyl ketone (Scheme 2, steps (i) - (iii))

To 2-ethylbutyric acid (2.09 g, 18 mmol) at O°C was added thionyl chloride (5.2 ml, 71.5 mmol) and the resulting mixture was allowed to warm to room temperature and stirred for 25 min. The mixture was diluted with dry THF then concentrated *in vacuo* at ambient temperature, azeotroping with further dry THF to remove last traces of SOCl₂. The residual oil was taken up in dry THF (10 ml) poured onto an ice-cold solution of CH₂N₂ (prepared from Diazald^{®} , 9 g, 42 mmol) in Et₂O and the resulting mixture allowed to warm to room temperature and stirred for 90 min. The reaction was quenched with AcOH (x 5), basified (5% KHCO₃) and extracted with EtOAc (x 3). The combined organic phases were washed with water, then sat. brine, filtered (Whatman^{®} 1 PS phase separator) and concentrated *in vacuo.* The crude oil was purified by flash chromatography on silica gel (eluant EtOAc: 40-60 pet. ether, 5:95 v/v) to give diazomethyl diethylmethyl ketone as a pale yellow liquid (261 mg, 1.86 mmol). To a solution of this diazoketone in EtOAc (10 ml) at room temperature was added a saturated solution of HBr in EtOAc portionwise until no more nitrogen was evolved. The reaction was basified (5% KHCO₃) and extracted with EtOAc (x 2). The combined organic phases were washed with water, sat. brine, filtered (Whatman^{®} 1 PS phase separator) and concentrated *in vacuo.* The crude material was purified by flash chromatography on silica gel (eluant gradient of EtOAc: 40-60 pet. ether, 4:96 to 10:90 v/v) to give the title ketone as a pale brown mobile oil (268 mg, 7.7%).
- NMR: (CDCl₃) (for the major rotamer 88.8%). δ 3.95 (2H, s); 1.56 - 1.46 (4H, m); 2.75 - 2.65 (1H, m); 0.91 - 0.85 (6H, m).

### 3B

### (3RS)-3-Benzyloxycarbonylamino-1-diethylmethylcarbonylmethyl-2,3-dihydro-5-phenyl-1H-1,4-benzodiazepin-2-one (Scheme 1, step (i))

This was prepared following the method of Reference Example 1B using the Bock benzodiazepine (3.56 mg, 0.925 mmol), sodium hydride (39 mg of 80% dispersion in oil, 1.3 mmol) and the bromomethylketone of Example 3A (268 mg, 1.4 mmol). The crude product was purified by flash chromatography on silica gel (eluant EtOAc: 40-60 pet. ether 25:85 v/v) to give the title benzodiazepine as a colourless oil (351 mg).
- R_{f}: (EtOAc: 40-60 pet. ether 40:60) 0.35.
- NMR: (CDCl₃) δ 7.64 - 7.13 (14H, m) ; 6.67 (1H, d, J = 8.25 Hz); 5.43 (1H, d, J = 8.25 Hz); 5.27 (2H, s); 5.15 (1H, d, J = 18 Hz); 4.78 (1H, d, J = 18 Hz); 2.45 - 2.35 (1H, m); 1.76 - 1.42 (4H, m); 0.90 - 0.85 (6H, m).

### 3C

### N-((3RS)-1-Diethylmethylcarbonylmethyl-2,3-dihydro-2-oxo-5-phenyl-1H-1,4-benzodiazepin-3-yl)-N'-(3-methylphenyl) urea. (Scheme 1, step (ii))

This was prepared following the method of Reference Example 1C using the benzodiazepine of Example 3B (351 mg, 0.68 mmol), 5% palladium-on-carbon (350 mg) and m-tolyl isocyanate (87 µl, 0.71 mmol). The crude product was purified by flash chromatography on silica gel (eluant EtOAc: 40-60 pet. ether 35:65 v/v) to give the title compound as a white solid (229 mg, 68%, >99% pure by HPLC).
- R_{f}: (EtOAc: 40-60 pet. ether 40:60) 0.24.
- NMR: (CDCl₃) δ 7.66 - 6.80 (15 H, m); 5.66 (1H, d, J = 8 Hz) ; 4.73 (1H, d, J = 18 Hz) ; 4.64 (1H, d, J = 18 Hz); 2.49 - 2.31 (1H, m); 2.26 (3H, s); 1.71 - 1.39 (4H, m); 0.86 - 0.81 (6H, m).
- M.S.: (FAB, +ve ion) m/e 497.3 (M+H).

### REFERENCE EXAMPLE 4

### N-((3RS)-1-Cyclopentylcarbonylmethyl-2,3-dihydro-2-oxo-5-phenyl-1H-1,4-benzodiazepin-3-yl)-N'-(3-methylphenyl) urea. Compound 4

### 4A

### Bromomethyl cyclopentyl ketone (Scheme 2, steps (i)- (iii))

This was prepared following the method of Example 3A. The intermediate diazoketone was prepared from cyclopentanecarboxylic acid (2.05 g, 18 mmol), thionyl chloride (5.2 ml, 72 mmol) and CH₂N₂ (generated from Diazald^{®} 9g, 42 mmol) and purified by flash chromatography on silica gel (eluant EtOAc: 40-60 pet. ether 15:85 v/v). The diazoketone was subsequently treated with a saturated solution of HBr in EtOAc. Flash chromatography on silica gel (eluant gradient EtOAc: 40-60 pet. ether 4:96 to 10:90 v/v) afforded the title ketone as a pale brown mobile oil (1.29 g, 37%).
- NMR: (CDCl₃) δ 3.99 (2H, s), 3.18 (1H, q, J = 8 Hz), 1.93 - 1.56 (8H, m).

### 4B

### (3RS)-3-Benzyloxycarbonylamino-1-cyclopentylcarbonylmethyl-2,3-dihydro-5-phenyl-1H-1,4-benzodiazepin-2-one. (Scheme 1, step (i))

This was prepared following the method of Reference Example 1B, using the Bock benzodiazepine (578 mg, 1.5 mmol), sodium hydride (63 mg of an 80% dispersion in oil, 2.1 mmol) and the bromomethylketone of Example 4A (431 mg, 2.25 mmol). The crude product was purified by flash chromatography on silica gel (eluant EtOAc: 40-60 pet. ether 30:70 v/v) to afford the title compound as a colourless crystalline solid (682 mg, 88.5%).
- R_{f}: (EtOAc: 40 - 60 pet. ether, 40:60) 0.25.
- NMR: (CDCl₃) δ 7.64 - 7.17 (14H, m); 6.60 (1H, d, J = 8.25 Hz); 5.42 (1H, d, J = 8.25 Hz); 5.14 (2H, s); 4.78 (1H, d, J = 17.8 Hz); 4.63 (1H, d, J = 17.8 Hz); 2.92(1H, q, J = 8 Hz); 1.85 - 1.55 (8H, m).

### 4C

### N-((3RS)-1-Cyclopentylcarbonylmethyl-2,3-dihydro-2-oxo-5-phenyl-1H-1,4-benzodiazepin-3-yl)-N'-(3-methylphenyl) urea. (Scheme 2, step (ii))

This was prepared following the method of Reference Example 1C using the benzodiazepine of Example 4B (680 mg, 1.32 mmol), 5% palladium-on-carbon (600 mg) and m-tolyl isocyanate (169 µl, 1.39 mmol). The crude product was purified by flash chromatography on silica gel (eluant EtOAc: 40-60 pet. ether 35:65 v/v) to give the title compound (463 mg, 71%).

The title compound was re-crystallised from hot methanol to give 161.8 mg of crystalline solid (>99% pure by HPLC).
- R_{f}: (EtOAc: 40-60 pet. ether, 40:60) 0.16.
- NMR: (CDCl₃) δ 7.66 - 6.80 (15H, m); 5.65 (1H, d, J = 8 Hz); 4.74 (1H, d, J = 18 Hz); 4.67 (1H, d, J = 18 Hz); 2.95 - 2.83 (1H, m); 2.28 (3H, s); 1.90 - 1.53 (6H, m).
- M.S.: (FAB +ve ion) m/e 495.2 (M+H).

### REFERENCE EXAMPLE 14

### N-((3RS)-1-Cyclopentylcarbonylmethyl-2,3-dihydro-2-oxo-5-(2-pyridyl)-1H-1,4-benzodiazepin-3-yl)-N'-(3-methylphenyl) urea. Compound 8.

### 14A

### (3RS)-3-Benzyloxycarbonylamino-1-cyclonentylcarbonylmethyl-2,3-dihydro-2-oxo-5-(2-pyridyl)-1H-1,4-benzodiazepin-2-one. (Scheme 1, step (i))

To a stirred solution of (3RS)-3-benzyloxycarbonylamino-2,3-dihydro-5-(2-pyridyl)-1H-1,4-benzodiazepin-2-one (R.M. Freidinger et al. Eur. Pat. No. 0 434 364 A2) (388 mg, 1 mmol) in DMF (5ml), cooled to -10°C under N₂, was added sodium hydride (42 mg of 80% dispersion in oil, 1.4 mmol). The mixture was stirred at -10°C for 30 min, when a solution of the bromomethyl ketone of Reference Example 4A (250 mg, 1.4 mmol) was added. The resulting mixture was allowed to warm to room temperature over 1 hr with stirring, then poured into dilute aqueous HCl (100 ml). The mixture was extracted twice with EtOAc and the organic extracts washed with brine, filtered (Whatman^{®} 1 PS phase separator) and concentrated in vacuo. The crude product was purified by flash chromatography on silica gel (eluant EtOAc/40-60 pet. ether 90:10 v/v) to give the title compound as a colourless solid (426 mg, 86%).
- R_{f}: (EtOAc) 0.35
- NMR: (CDCl₃) δ 8.62 (1H, d, J = 8 Hz) , 8.17 (1H, d, J = 8 Hz), 7.78 (1H, t, J = 8 Hz), 7.50 (1H, t, J = 8 Hz), 7.4 - 7.15 (9H, m), 6.76 (1H, d, J = 8.5 Hz), 5.51 (1H, d, J = 8.5 Hz), 5.16 (2H, m), 4.80 (1H, d, J = 17.5 Hz), 4.42 (1H, d, J=17.5 Hz), 2.92 (1H, m), 1.9 - 1.5 (8H, m).

### 14B

### N-((3RS)-1-Cyclopentylcarbonylmethyl-2,3-dihydro-2-oxo-5-(2-pyridyl)-1H-1,4-benzodiazepin-3-yl)-N'-(3-methylphenyl) urea. (Scheme 1, step (ii))

The benzodiazepine of Reference Example 14A (426 mg, 0.86 mmol) was treated with 40% HBr in acetic acid (6 ml) and the mixture stirred at room temperature for 2 hrs. The mixture was azeotroped twice with toluene and the residue partitioned between EtOAc and 1 M NaOH. The organic portion was filtered (Whatman^{®} 1 PS phase separator), evaporated, taken up in CH₂Cl₂ (5 ml) and treated with m-tolyl isocyanate (130 µl, 0.95 mmol) at room temperature with stirring for 2 hrs. The mixture was evaporated and chromatographed (eluant EtOAc/40-60 pet. ether 70:30 v/v) to provide a yellow solid which was recrystallised from acetonitrile to give the colourless product (73 mg, 17%).
- R_{f}: (EtOAc/Hexane, 60:40 v/v) 0.38
- NMR: (CDCl₃) δ 8.6 (1H, d, J = 6 Hz, 7.95 (1H, d, J= 6 Hz), 7.6 - 7.0 (10H, m), 6.80(1H, d, J = 8.5 Hz), 5.78 (1H, d, J = 8.5 Hz), 5.10 (1H, d, J = 14 Hz), 5.00 (1H, d, J = 14 Hz), 2.82 (1H, m), 2.14 (3H, s), 1.90 - 1.50 (8H, m).
- M.S.: (FAB, +ve ion) m/e. 496.3 (M+H)

### REFERENCE EXAMPLE 16

### N-((3RS)-1-Cyclopentylcarbonylmethyl-2,3-dihydro-2-oxo-5-phenyl-1H-1,4-benzodiazepin-3-yl)-N'-(3-carboxyphenyl) urea. Compound 11

### 16A

### (3RS)-3-p-Nitrobenzyloxycarbonylamino-1-cyclopentylcarbonylmethyl-2,3-dihydro-2-oxo-5-phenyl-1H-1,4-benzodiazepin-2-one. (Scheme 3. step (i))

The benzodiazepine of Reference Example 4B (2.04 g, 4.13 mmol) was hydrogenated over 5% palladium-on-carbon catalyst (1.2g) as described in Reference Example 1C. The amine was taken up in dry THF (15 ml) and triethylamine (0.626 ml, 4.5 mmol) and the solution cooled to O°C. The stirred mixture was treated with p-nitrophenyl chloroformate (0.91 g, 4.5 mmol) and stirred at room temperature for 1 hr, then evaporated and flash chromatographed (eluant EtOAc/Hexane 40:60 v/v) to provide the product as a yellow solid (675 mg, 32%).
- R_{f}: (EtOAc/Hexane, 60:40 v/v) 0.52
- NMR: (CDCl₃) δ 8.2 (2H, d, J = 8 Hz), 7.6 - 7.0 (12H, m), 5.42 (1H, d, 8.5 Hz), 4.82 (1H, d, J = 16.5 Hz), 4.62 (1H, d, J = 16.5 Hz), 2.97 (1H, m), 1.9 - 1.4 (8H, m).

### 16B

### N-((3RS)-1-cyclopentylcarbonylmethyl-2,3-dihydro-2-oxo-5-phenyl-1H-1,4-benzodiazepin-3-yl)-N'-(3-carboxyphenyl) urea. (Scheme 3, step (ii))

To a stirred solution of the benzodiazepine of Reference Example 16A (175 mg, 0.333 mmol) in DMF (5 ml) was added m-aminobenzoic acid (72 mg, 0.52 mmol) and triethylamine (125 µl). The mixture was stirred at 45°C for 18 hrs, cooled and diluted with EtOAc. The mixture was washed with 0.3 M HCl and brine, then filtered (Whatman^{®} 1 PS phase separator) and evaporated. The residue was flash chromatographed (eluant EtOAc/Hexane/acetic acid 60:40:2 v/v/v) to provide the title compound as a colourless solid (132 mg, 76%).
- R_{f}: (EtOAc/Hexane/acetic acid 60:40:2 v/v/v) 0.25
- NMR: (CDCl₃) δ 8.41 (1H, s), 8.37 (1H, d, J = 7.5 Hz), 8.16 (1H, d, J = 7.5 Hz), 7.82 (1H, s), 7.7 - 7.2 (12H, m), 5.65 (1H, d, J = 8.5 Hz), 5.81 (1H, d, J = 15 Hz), 4.66 (1H, d, J = 15 Hz), 2.95 (1H, m), 1.95 - 1.5 (8H, m).
- M.S.: (FAB, +ve ion) m/e 419 (M+Na - H₂NC₆H₄CO₂H)

### REFERENCE EXAMPLE 17

### N-((3R)-1-Cyclopentylcarbonylmethyl-2,3-dihydro-2-oxo-5-phenyl-1H-1,4-benzodiazepin-3-yl)-N'-(3-methylphenyl) urea. Compound 12

The benzodiazepine of Reference Example 4B (1.13 g, 2.3 mmol) was hydrogenated as described in Reference Example 4C and the amine chromatographed (eluant CHCl₃/MeOH/AcOH 25:2:1) to provide a yellow oil. This was taken up in acetonitrile and (S)-mandelic acid (335 mg, 2.20 mmol) added to the stirred solution, followed 30 min later by 3,5-dichlorosalicylaldehyde (10 mg). After stirring overnight the resultant precipitate was collected by suction filtration and washed with cold acetonitrile, to give a white solid (680 mg, 59%).

The solid (460 mg, 0.897 mmol) was partitioned between CHCl₃ and 0.5 M NaOH. The organic portion was washed with brine, filtered (Whatman^{®} 1 PS phase separator) and evaporated. The residue was taken up in CH₂Cl₂ (5 ml) and treated with m-tolyl isocyanate (110 µl, 0.852 mmol) at room temperature for 1 hr. The mixture was evaporated and chromatographed (eluant EtOAc/Hexane 40:60 v/v) to provide a colourless solid (320 mg, 76%).
- R_{f}: (EtOAc/Hexane 40:60 v/v) 0.16
[α]_{D} = +100.4° (CHCl₃, C = 0.96)
NMR and M.S. identical to Example 4C.

### EXAMPLE 26

### N-((3R)-1-tert-Butylcarbonylmethyl-2,3-dihydro-2-oxo-5-phenyl-1H-1,4-benzodiazepin-3-yl)-N'-(3-methyl-phenyl) urea. Compound 38

The benzodiazepine of Example 2B was hydrogenated and the resultant amine resolved into its (R)-isomer (S)-mandelate salt as described in Reference Example 17. A portion of the salt (150 mg, 0.299 mmol) was partitioned between CHCl₃ and 0.25 M NaOH. The organic portion was washed with brine, filtered (Whatman^{®} 1 PS paper) and evaporated. The residue was taken up in CH₂Cl₂ and m-tolyl isocyanate (42 µl, 0.33 mmol) added. The mixture was stirred at room temperature for 1 hr, then evaporated and chromatographed (eluant EtOAc/hexane, 40/60, v/v) to provide a white solid which was freeze dried from acetonitrile/water (125 mg, 87%).

Data identical to Example 2C.

### EXAMPLE 27

### N-((3RS)-1-tert-Butylcarbonylmethyl-2,3-dihydro-2-oxo-5-(2-pyridyl)1-1H-1,4-benzodiazepin-3-yl)-N'-(3-methylphenyl) urea. Compound 39

### 27A

### (3RS)-3-Benzyloxycarbonylamino-1-tert-butylcarbonylmethyl-2,3-dihydro-2-oxo-5-(2-pyridyl)-1H-1,4-benzodiazepin-2-one (Scheme 1, step (ii))

(3RS)-3-Benzyloxycarbonylamino-2,3-dihydro-5-(2-pyridyl)-1H-1,4-benzodiazepin-2-one (R.M. Freidinger et al. Eur. Pat. No. 0 434 364 A2) (2.02 g, 5 mmol) was alkylated with 1-bromopinacolone (1.08 g, 6 mmol) as described in Reference Example 14A, to provide a white solid after chromatography (eluant EtOAc/hexane, 80:20 v/v) (2.16 g, 86%).

### 27B

### N-((3RS)-1-tert-Butylcarbonylmethyl-2,3-dihydro-2-oxo-5-(2-pyridyl)-1H-1,4-benzodiazepin-3-yl)-N'-(3-methylphenyl) urea (Scheme 1, step (ii))

A portion of the benzodiazepine from Example 27A (502 mg, 1 mmol) was taken up in CH₂Cl₂ (2 ml) at -70°C and treated dropwise with BBr₃ (6.5 ml, 1.0 M solution in CH₂Cl₂) and the mixture stirred for 3 hrs, during which time the cold bath expired. The mixture was quenched with water, then partitioned between EtOAc and 1M NaOH. The organic portion was washed with brine, filtered (Whatman^{®} 1 PS paper) and evaporated. The residue was taken up in CH₂Cl₂ (3 ml) and treated with m-tolyl isoyanate (135 µl, 1.05 mmol) at room temperature for 1 hr. The mixture was evaporated and chromatographed (EtOAc/Hexane 75:25 v/v) and the resultant white solid recrystallised from acetonitrile (180 mg, 38%).
- R_{f}: (EtOAc/Hexane, 60:40 v/v) 0.28.
- ¹H NMR: (CDCl₃) δ 8.78 (d, 1H, J = 2 Hz) ; 8.27 (d, 1H, J = 7 Hz); 7.95 (m, 1H); 7.65-6.9 (m, 11H); 5.83 (d, 1H, J = 8 Hz); 5.10 (d, 1H, J = 16 Hz); 4.70 (d, 1H, J 16 Hz); 2.42 (s, 3H); 1.40 (s, 9H).
- M.S.: (FAB) (M+H)⁺ 484.4

### EXAMPLE 28

### N-((3RS)-1-tert-Butylcarbonylmethyl-2,3-dihydro-2-oxo-5-(phenyl-1H-1,4-benzodiazepin-3-yl)-N'-(3-carboxylphenyl) urea. Compound 40

The benzodiazepine of Example 2B was hydrogenated as described in Reference Example 1C and the resultant amine (600 mg, 1.72 mmol taken up in dry THF (8 ml) and Et₃N (260 µl, 1.9 mmol). A solution of p-nitrophenyl chloroformate (0.38 g, 1.9 mmol) in THF (3 ml) was added dropwise and the mixture stirred at room temperature for 1 hr, evaporated and chromatographed (eluant EtOAc/hexane, 40:60, v/v) to give a white solid (670 mg). This solid was taken up in DMF (10 ml) and treated with m-amino benzoic acid (245 mg, 1.75 mmol) at 45°C for 18 hrs. The mixture was evaporated and chromatographed (eluant EtOAc/hexane/acetic acid, 60:40:2, v/v/v) and the product recrystallised from acetonitrile to provide the title compound (328 mg, 38%).
- ¹H NMR: CDCl₃) δ 7.8 - 7.0 (14H, m); 6.80 (1H, d, J = 7 Hz); 5.6 (1H, d, J = 8 Hz); 4.9 (1H, d, J = 18 Hz); 4.8 (1H, d, J = 18 Hz); 1.40 (9H, s).
- M.S.: (FAB) (M+H)⁺ = 513.4

### REFERENCE EXAMPLE 29

### N-((3RS)-1-Cyclopentylcarbonylmethyl-2,3-dihydro-2-oxo-5-phenyl-1H-1,4-benzodiazepin-3-yl)-N'-(6-methyl-2-pyridyl) urea. Compound 27

This was prepared following the method of Reference Example 1C using the benzodiazepine of Reference Example 4B (165 mg, 0.33 mmol), 5% palladium-on-carbon (80 mg) and the isocyanate prepared below:

To a stirred solution of 2-amino-6-picoline (108 mg, 1 mmol) in CH₂Cl₂ (10 ml) at -20°C was added triphosgene (110 mg, 0.37 mmol) and pyridine (79 mg, 1 mmol). The mixture was stirred for 30 min while warming to room temperature. Pyridine (79 mg, 1 mmol) was added and the reaction was heated to reflux for 30 min. The resulting solution of isocyanate was cooled to O°C and used directly.

The crude product was purified by flash chromatography on silica gel (Eluant EtOAc: 40-60 pet ether 80-20 v/v) to give the title compound as a white solid (45 mg, 28%, 96% pure by HPLC).
- R_{f}: (EtOAc: 40-60 pet ether 20:80) 0.20.
- NMR: (CDCl₃) δ 10.8 (1H, broads); 7.70 - 6.80 (13H, m); 5.70 (1H, d, J =8 Hz); 4.82 (1H, d, J = 17 Hz); 4.75 (1H, d, J = 17 Hz); 2.95 (1H, quintet, J =7 Hz); 2.60 (3H, s); 1.95 - 1.75 (4H, m); 1.70 - 1.45 (4H, m).
- M.S.: (FAB, +ve ion) m/e 496.0 (M+H).

### REFERENCE EXAMPLE 30

### N-((3RS)-1-Cyclopentylcarbonylmethyl-2,3-dihydro-2-oxo-5-phenyl-1H-1,4-benzodiazepin-3-yl)-N'-(3-pyridyl) urea. Compound 26

This was prepared following the method of Reference Example 1C using the benzodiazepine of Reference Example 4B (270 mg, 0.55 mmol), 5% palladium-on-carbon (100 mg) and the isocyanate prepared below:

To a stirred solution of nicotinic acid (172 mg, 1.4 mmol) and diisopropylethylamine (250 µl, 1.4 mmol) in THF (15 ml) at O°C was added isobutyl chloroformate (182 µl, 1.4 mmol). The mixture was stirred at O°C for 30 min, then a solution of sodium azide (97 mg, 1.5 mmol) in H₂0 (1 ml) was added and the mixture was stirred for 2 hours while warming to room temperature. The mixture was evaporated *in vacuo* and the residue taken up in cold EtOAc, washed with cold saturated KHCO₃ and cold brine, filtered (Whatman^{®} 1 PS phase separator) and concentrated *in vacuo.* The residue was heated in THF (5 ml) at 60°C for 5 min to give the isocyanate and used directly.

The crude product was purified by flash chromatography on silica gel (Eluant CHCl₃: MeOH: AcOH 100:2:1 v/v) to give the title compound as a crystalline solid (34 mg, 13% > 99% pure by HPLC).
- R_{f}: (CHCl₃: MeOH: AcOH, 100:2:1) 0.10.
- NMR: (CDCl₃) δ 8.70 - 7.20 (16H, m); 5.50 (1H, d, J = 8 Hz); 4.75 (1H, d, J =18Hz); 4.67 (1H, d, J = 18 Hz); 3.70 (1H, m), 1.90 - 1.20 (8H, m).
- MS: (FAB, +ve ion) ²/ₘ 482.0 (M+H)

### REFERENCE EXAMPLE 31

### N-((3RS)-1-Cyclopentylcarbonylmethyl-2,3-dihydro-2-oxo-5-phenyl-1H-1,4-benzodiazepin-3-yl)-N'-(3-carboxymethylphenyl) urea. Compound 24

### 31A

### Methyl 3-amino phenyl acetate

To a solution of 3-aminophenylacetic acid (3.02 g, 20 mmol) and methanol (60 ml) was added acetyl chloride (2 ml, 28 mmol). The reaction was heated to reflux for six hours. After allowing reaction to cool to room temperature the solvent was evaporated *in vacuo.* The resultant product was taken up in CHCl₃ and washed with saturated KHCO₃ and brine, filtered (Whatman^{®} 1 PS phase separator), and concentrated *in vacuo* to afford the title acetate as a brown mobile oil (3.0 g, 91%).
- NMR: (CDCl₃) δ 7.35 - 6.90 (4H, m); 4.75 (2H, s); 3.80 (3H, s); 3.65 (2H, s).

### 31B

### N-((3RS)- 1-Cyclopentylcarbonylmethyl-2,3-dihydro-2-oxo-5-phenyl-1H-1,4-benzodiazepin-3-yl)-N'-(3-methoxycarbonylmethylphenyl) urea. (Scheme 1, step (ii)

This was prepared following the method of Reference Example 1C using the benzodiazepine of Reference Example 4B (165 mg, 0.33 mmol), 5% palladium-on-carbon (80 mg) and the isocyanate prepared from the amine of Reference Example 31A following the method of Reference Example 29.

The crude product was purified by flash chromatography on silica gel (eluant EtOAc: hexane fr 60:40 v/v) to afford the methyl ester as a white solid (180 mg, 97 %).
- R_{f}: (EtOAc: hexane fr 60:40) 0.23.

### 31C

### N-((3RS)-1-cyclopentylcarbonylmethyl-2,3-dihydro-2-oxo-5-phenyl-1H-1,4-benzodiazepin-3-yl)-N'-(3-carboxymethylphenyl) urea

To a solution of the benzodiazepine of Reference Example 31B (180 mg, 0.32 mmol) in dioxan (10 ml) was added a solution of LiOH.H₂O (27 mg, 0.64 mmol) in H₂0 (6 ml). The mixture was stirred for 18 hours, then dilute aq. HCl (10 ml) was added. The mixture was extracted with EtOAc twice, and the combined organic layers were washed with water and brine, filtered (Whatman^{®} 1 PS phase separator) and concentrated *in vacuo*. The crude product was purified by flash chromatography on silica gel (eluant EtOAc: hexane fr: AcOH 70:30:2 v/v/v) to give the title compound as a white solid (105 mg, 61%, > 98% pure by HPLC).
- R_{f}: (EtOAc: hexane fr: AcOH, 70:30:2) 0.20
- NMR: (CDCl₃) δ 8.00 (1H, s); 7.65 - 6.90 (15H, m); 5.65 (1H, d, J = 8 Hz); 4.71 (2H, s); 3.50 (2H, s); 1.95 - 1.50 (9H, m).
- M.S.: (FAB, +ve ion) m/e 539.1 (M+H).

### EXAMPLE 36

### N-((3RS)-1-tert-Amylcarbonylmethyl-2,3-dihydro-2-oxo-5-phenyl-1H-1,4-benzodiazepin-3yl)-N'-(3-methylphenyl) urea. Compound 41

### 36A

### 1-Bromo-3,3-dimethylpentan-2-one (Scheme 2, steps (i) (iii))

This was prepared following the method of Example 3A. The intermediate diazoketone was prepared from 2,2 dimethylbutyric acid (5.8 g, 50 mmol), thionyl chloride (14.6 ml, 200 mmol) and CH₂N₂ (generated from Diazald^{®} 21.5 g, 100 mmol) and purified by flash chromatography on silica gel (eluant EtOAc: hexane fr 20:80 v/v). The diazoketone was subsequently treated with a saturated solution of HBr in EtOAc. Flash chromatography on silica gel (Eluant EtOAc: hexane fr 15:85 v/v) afforded the title ketone as a pale brown oil (1.50 g, 16%).
- NMR: (CDCl₃) δ 4.25 (2H, s); 1.70 (1H, q, J = 8 Hz) ; 1.30 (6H, s); 0.97 (3H, t, J = 8 Hz).

### 36B

### N-((3RS)-1-tert-Amylcarbonylmethyl-3-benzyloxy-carbonylamino-2,3-dihydro-5-phenyl-1H-1,4-benzodiazepin-2-one. (Scheme 1, step (i))

This was prepared following the method of Reference Example 1B, using the Bock benzodiazepine (500 mg, 1.25 mmol), sodium hydride (54 mg of an 80% dispersion in oil, 1.75 mmol) and the bromomethyl ketone of Example 36A (300 mg, 1.5 mmol). The crude product was purified by flash chromatography on silica gel (eluant EtOAc: hexane fr 35:65 v/v) to afford the title compound as a colourless crystalline solid (565 mg, 91%).
- R_{f}: (EtOAc: hexane fr 40:60) 0.20
- NMR: (CDCl₃) δ 7.90 - 7.30 (14H, m); 6.90 (1H, d, J = 8 Hz); 5.80 (1H, d, J =8 Hz); 5.40 (2H, s); 5.10 (1H, d, J =18 Hz); 4.75 (1H, d, J =18 Hz); 1.90 (2H, m); 1.40 (6H, s); 1.00 (3H, t, J = 8 Hz).

### 36C

### N-((3RS)-1-tert-Amylcarbonylmethyl-2,3-dihydro-2-oxo-5-phenyl-1H-1,4-benzodiazepin-3-yl)-N'-(3-methylphenyl) urea. (Scheme 1, step (ii))

This was prepared following the method of Example 1C using the benzodiazepine of Example 36B (240 mg, 0.46 mmol), 5% palladium-on-carbon (250 mg) and m-tolyl isocyanate (76 µl, 0.6 mmol). The crude product was purified by flash chromatography on silica gel (eluant EtOAc: hexane fr 40:60 v/v) to give the title compound which was crystallised from acetonitrile to give a white solid (98 mg, 43%, >99% by HPLC).
- R_{f}: (EtOAc: hexane fr 40:60) 0.20.
- NMR: (CDCl₃) δ 7.50 - 6.80 (15H, m); 5.65 (1H, d, J = 8 Hz); 4.70 (1H, d, J = 18 Hz); 4.65 (1H, d, J = 18 Hz); 2.20 (3H, s); 1.50 (2H, q, J = 8 Hz); 1.00 (6H, s); 0.70 (3H, t, J = 8 Hz).
- M.S.: (FAB +ve ion) m/e 497.2 (M+H).

### EXAMPLE 37

### N-((3RS)-1-tert-Amylcarbonylmethyl-2,3-dihydro-2-oxo-5-phenyl-1H-1,4-benzodiazepin-3-yl)-N'-(3-carboxyphenyl) urea. Compound 42

### 37A

### (3RS)-3-p-Nitrobenzyloxycarbonylamino-1-tert-amylcarbonylmethyl-2,3-dihydro-2-oxo-5-phenyl-1H-1,4-benzodiazepin-2-one (Scheme, step(i))

This was prepared following the method of Reference Example 16A using the benzodiazepine of Example 36B (325 mg, 0.68 mmol), 5% palladium-on-carbon (250 mg) and p-nitrophenyl chloroformate (150 mg, 0.75 mmol). The crude product was purified by flash chromatography on silica gel (eluant EtOAc: hexane fr 35:65 v/v) to provide the product as a yellow solid (135 mg, 38%).
- R_{f}: (EtOAc: hexane fr 40:60) 0.28.
- NMR: (CDCl₃) δ 8.10 (2H, d, J =8 Hz); 7.50 - 7.00 (12H, m); 5.40 (1H, d, J=8 Hz); 4.85 (1H, d, J =17 Hz); 4.60 (1H, d, J =17 Hz); 1.55 (2H, q, J = 8 Hz); 1.10 (6H, s); 0.75 (3H, t, J = 8 Hz).

### 37B

### N-((3RS)- 1-tert-Amylcarbonylmethyl-2,3-dihydro-2-oxo-5-phenyl-1H-1,4-benzodiazepin-3-yl)-N'-3-(carboxyphenyl) urea (Scheme 3, step (ii))

This was prepared following the method of Reference Example 16B using the benzodiazepine of Example 37A, (135 mg, 0.26 mmol) and m-aminobenzoic acid (54 mg, 0.39 mmol). The crude product was purified by flash chromatography on silica gel (eluant EtOAc: hexane fr: AcOH 60:40:2) to give the title compound which was crystallised from acetonitrile to give a colourless solid (30 mg, 22%, > 97% pure by HPLC).
- R_{f}: (EtOAc: hexane fr: AcOH 60:40:2) 0.20.
- NMR: (CDCl₃) δ 8.10 - 6.80 (16H, m) ; 5.50 (1H, d, J = 8 Hz); 4.80 (1H, d, J = 18 Hz); 4.65 (1H, d, J = 18 Hz); 1.40 (2H, q, J = 8 Hz); 1.00 (6H, s); 0.70 (3H, t, J = 8 Hz).
- M.S.: (FAB +ve ion) m/e 527.1 (M+H).

### EXAMPLE 38

### N-((3RS)-1-tert-Butylcarbonylmethyl-2,3-dihydro-2-oxo-5-(2-pyridyl)-1H-1,4-benzodiazepin-3-yl)-N'-(3-dimethyl-aminophenyl) urea. Compound 43

This was prepared following the method of Example 27B using the benzodiazepine of Example 27A (185 mg, 0.38 mmol), BBr₃ (2.5 ml, 1.0 M sol. in CH₂Cl₂), and the isocyanate prepared as below:

To a solution of 3-dimethylaminobenzoic acid (165 mg, 1 mmol) in toluene (5 ml) was added diphenylphosphoryl azide (275 mg, 1 mmol) and Et₃N (101 mg, 1 mmol). The mixture was stirred at room temperature for 2 hrs, then heated at reflux for 3 hrs. The solution of the above free amine in toluene (5 ml) was added to the cooled mixture and stirred at room temperature overnight, then evaporated, taken up in EtOAc, washed with 5% KHCO₃, H₂0 and brine, filtered (Whatman^{®} 1 PS phase separator) and evaporated, and chromatographed (eluant EtOAc) to provide a colourless solid (38 mg, 20%, 91% pure by HPLC).
- R_{f}: (EtOAc) 0.15.
- NMR: (CDCl₃) δ 8.78 (d, 1H, J = 2 Hz); 8.27 (d, 1H, J = 8 Hz); 7.95 (m, 1H); 7.65 - 6.6 (m, 11H); 5.83 (d, 1H, J = 8 Hz); 5.05 (d, 1H, J = 16 Hz); 4.60 (d, 1H, J=16 Hz); 3.00 (s, 6H); 1.40 (s, 9H).
- M.S.: (FAB) (M+H)⁺

### EXAMPLE 39

### N-((3RS)-1-tert-Butylcarbonylmethyl-2,3-dihydro-2-oxo-5-(2-pyridyl)-1H-1,4-benzodiazepin-3-yl)-N'-(3-carboxyphenyl) urea. Compound 44

### 39A

### mono-Methyl isophthalate

Dimethyl isophthalate (2.28 g, 11.7 mmol) was taken up in dioxan (25 ml) and treated with a solution of LiOH.H₂0 (510 mg, 12.1 mmol) in water (15 ml) as described in Reference Example 31B. The product was purified by chromatography (eluant EtOAc: hexane fr:AcOH 50:50:2 v/v/v) to provide the title compound (1.1 g, 48%).
- NMR: (CDCl₃) δ 8.45 (s, 1H); 8.10 (2H, m); 7.45 (1H, m); 3.80 (3H, s).

### 39B

### N-((3RS)-1-tert-Butylcarbonylmethyl-2,3-dihydro-2-oxo-5-(2-pyridyl)-1H-1,4-benzodiazepin-3-yl)-N'-(3-methoxycarbonylphenyl) urea. (Scheme 1, step (ii))

This was prepared following the method of Example 27B using the benzodiazepine of Example 27A (185 mg, 0.38 mmol), BBr₃ (2.5 ml, 1.0 M sol. in CH₂Cl₂) and the isocyanate prepared using the acid of Example 39A (250 mg, 1.4 mmol) with following the method of Example 38. The product was purified by chromatography (eluant EtOAc) to provide the title compound (110 mg, 56%).
- NMR: (CDCl₃) δ 8.30 (1H, d, J = 2 Hz); 7.95 - 7.00 (13H, m); 5.60 (1H, d, J = 8 Hz); 4.80 (1H, d, J = 16 Hz); 4.50 (1H, d, J = 16 Hz); 3.70 (3H, s); 1.20 (9H, S).

### 39C

### ((3RS)-1-tert-Butylcarbonylmethyl-2,3-dihydro-2-oxo-5-(2-pyridyl)-1H-1,4-benzodiazepin-3-yl)-N'-(3-carboxyphenyl) urea

This was prepared following the method of Reference Example 31B, using the benzodiazepine of Example 39B (110 mg, 0.21 mmol) and LiOH.H₂0 (17 mg, 0.42 mmol). The crude product was purified by flash chromatography (eluant CHCl₃: MeOH: AcOH 75:2:1 v/v/v) to provide the title compound which was freeze-dried from MeCN/H₂0 to give the product as a white solid (20 mg, 19%, >98% pure by HPLC).
- R_{f}: (EtOAc:AcOH 100:2) 0.12.
- NMR: (CDCL₃) δ 8.50 (1H, d, J =2 Hz); 8.10 - 7.35
- M.S.: (13H, m); 5.65 (1H, s); 5.15 (1H, d, J = 18 Hz); 4.90 (1H, d, J = 18 Hz); 1.35 (9H, s). (FAB) (M+H)+

### EXAMPLE 41

### N-((3RS)-1-tert-Butylcarbonylmethyl-2,3-dihydro-2-oxo-5-phenyl-1H-1,4-benzodiazepin-3-yl)-N'-(3-dimethyl-aminophenyl) urea. Compound 46

This was prepared following the method of Reference Example 1C using the benzodiazepine of Example 2B (380 mg, 0.8 mmol), 5% palladium-on-carbon (300 mg) and the isocyanate (3 mmol) prepared as in Example 38.

The crude product was purified by flash chromatography on silica gel (eluant EtOAc:hexane fr 55:45 v/v) and crystallised from acetonitrile to give the title compound as a white powder (127 mg, 31 %, >99% pure by HPLC).
- R_{f}: (EtOAc:hexane fr 60:40) 0.23.
- NMR: (CDCl₃) δ 7.60 - 6.9 (15H, m); 5.60 (1H, d, J = 8 Hz); 4.78 (1H, d, J - 18 Hz); 4.68 (1H, d, J = 18 Hz); 2.80 (6H, s); 1.20 (9H, s).
- M.S.: (FAB, +ve ion) m/e 512.5 (M+H).

### EXAMPLE 43

### N-((3RS)-1-tert-Butylcarbonylmethyl-2,3-dihydro-2-oxo-5-phenyl-1H-1,4-benzodiazepin-3-yl)-N'-(3-methoxyphenyl) urea. COMPOUND 48

This was prepared following the method of Reference Example 1C using the benzodiazepine of Example 2B (385mg, 0.8 mmol), 5% palladium-on-carbon (300 mg) and 3-methoxyphenyl isocyanate (136 µl, 1.04 mmol). The crude product was purified by flash chromatography on silica gel (eluant EtOAc: hexane fr 45:55 v/v) and crystallised from acetonitrile to provide the title compound as a white powder (249 mg, 63%, >99% pure by HPLC).
- R_{f}: (EtOAc: hexane fr 50:50) 0.23.
- NMR: (CDCl₃) δ 7.60 - 6.50 (15H, m); 5.85 (1H, d, J = 8 Hz); 4.92 (1H, d, J = 18 Hz); 4.82 (1H, d, J = 18 Hz); 3.90 (3H, s); 1.30 (9H, s).
- M.S.: (FAB, +ve ion) m/e

### EXAMPLE 45

### N-((3RS)-1-tert-Butylcarbonylmethyl-2,3-dihydro-2-oxo-5-phenyl-1H-1,4-benzodiazepin-3-yl)-N'-(3-nitrophenyl) urea. Compound 50

This was prepared following the method of Reference Example 1C using the benzodiazepine of Example 2B (385mg, 0.8 mmol), 5% palladium-on-carbon (300 mg) and 3-nitrophenyl isocyanate (171 mg, 1.04mmol). The crude product was purified by flash chromatography on silica gel (EtOAc: hexane Fr 45:55 v/v) and crystallised from acetonitrile to provide the title compound as a pale yellow solid (283 mg, 69% >99% pure by HPLC).
- R_{f}: (EtOAc: hexane fr 50:50) 0.24.
- NMR: (CDCl₃) δ 8.10 - 6.90 (15H, m); 5.65 (1H, d, J = 8 Hz); 4.85 (2H, s); 1.25 (9H, S).
- M.S.:

### EXAMPLE 47

### N-((3RS)-1-tert-Butylcarbonylmethyl-2,3-dihydro-2-oxo-5-phenyl-1H-1,4-benzodiazepin-3-yl)-N'-(3-formylaminophenyl) urea. Compound 51

### 47A

### 3-Formylaminobenzoic acid

Acetic anhydride (50 ml) was added to 98% formic acid (85 ml). The mixture was stirred at room temperature for 30 mins, then 3-aminobenzoic acid (10 g, 73 mmol) was added and the mixture was stirred for one hour at room temperature. Water (850 ml) was added and the mixture was stirred ovenight at room temperature. The resultant white precipitate was collected (10.66 g, 88%).
- NMR: (CDCl₃) δ 8.90 - 7.50 (7H, m).

### 47B

### N-((3RS)-1-tert-Butylcarbonylmethyl-1-2,3-dihydro-2-oxo-5-phenyl-1H-1,4-benzodiazapin-3-yl)-N'-(3-formylaminophenyl) urea (Scheme 1 step (ii))

The benzodiazepine of Example 2B was hydrogenated as described in Reference Example 1C and the resultant amine (250 mg, 0.71 mmol) in toluene (2 ml) was treated with the isocyanate prepared using the acid of Example 47A (410 mg, 2.5 mmol) following the method of Example 38. The product was purified by chromatography twice (eluant EtOAc and CHCl₃:MeOH:AcOH 80:20:1 v/v/v) and crystallised from acetonitrile to give a colourless solid (75 mg, 21%, >97% pure by HPLC).
- ¹H NMR: (CDCl₃) δ 8.90 - 8.10 (3H, m); 7.75 - 7.10 (14½H, m); 6.70 (1/2H, d, J=18Hz); 5.75 (1H, m); 4.90 (1H, m); 1.30 (9H, 3s).
- M.S.: [M+H]⁺ = 512.2.

### EXAMPLE 48

### N-((3R)-1-((2R)-2-Hydroxy-3,3-dimethylbutyl)-2,3-dihydro-2-oxo-5-phenyl-1H-1,4-benzodiazepin-3-yl)-N'-(3-methylphenyl) urea. Compound 52

The benzodiazepine urea of Example 26 (180 mg, 0.373 mmol) was taken up in a mixture of methanol and dichloromethane (5 ml, 1/1, v/v) and CeCl₃.7H₂0 (155 mg, 0.416 mmol) was added and the mixture stirred to dissolve all solids and then cooled to - 78°C. Sodium borohydride (20 mg, 0.529 mmol) was added and the mixture stirred at -78°C for 10 min then at -10°C for 1h, warming to room temp over 2 hours. The mixture was partitioned between CHCl₃ and brine and the organic portion dried and evaporated. The residue was purified by flash chromatography on silica gel (eluant EtOAc:hexane fr 40:60 v/v) to provide a colourless solid (105 mg, 58%, >98% pure by HPLC).
- R_{f}: (EtOAc: 60- 80 pet. ether 40:60) 0.31
- ¹H NMR: (CDCl₃) δ 7.8 - 6.9 (15H, m), 5.6 (1H, d, J = 8Hz); 4.35 (1H, m); 3.6 (2H, m); 2.2 (3H, s); 0.85 (9H, s).
- M.S.: (FAB, +ve ion) m/e = 485.3 [M+H]

### EXAMPLE 50

### N-((3RS)-1-tert-Butylcarbonylmethyl-2,3-dihydro-2-oxo-5-phenyl-1H-1,4-benzodiazepin-3-yl)-N'-(3-cyanophenyl) urea. Compound 23

This was prepared following the method of Reference Example 1C using the benzodiazepine of Example 2B (290 mg, 0.6 mmol), 5% palladium-on-carbon (150 mg) and the isocyanate prepared from 3-cyanobenzoic acid (295 mg, 2 mmol) following the method of Example 30. The crude product was purified by flash chromatography on silica gel (eluant CHCl₃:AcOH, 100:2 v/v) to afford the title compound which was lyophilised from MeCN/H₂0 (36 mg, 12%, >96% pure by HPLC).
- NMR: (CDCl₃) δ 8.20 - 8.05 (4H, m); 7.70 - 7.10 (11H, m); 5.50 (1H, d, J = 8Hz); 4.90 (1H, d, J = 17Hz); 4.70 (1H, d, J = 17Hz); 1.15 (9H, s).
- M.S.: (FAB): [M+H]⁺ = 494.2

### EXAMPLE 54

### N-((3RS)-1-tert-Butylcarbonylmethyl-2,3-dihydro-2-oxo-5-(2-chlorophenyl-1H-1,4-benzodiazepin-3-yl)-N'-(3-methylphenyl) urea. Compound 55

### 54A

### (3RS)-3-Benzyloxycarbonylamino-1-tert-butylcarbonylmethyl-7-chloro-5-(2-chlorophenyl)-1H-1,4 benzodiazepin-2-one (Scheme 1. step (1))

This was prepared following the method of Reference Example 1B, using (3RS)-3-benzyloxy-carbonylamino-7-chloro-5-(2-chlorophenyl)-1H-1,4-benzodiazepin-2-one (241 mg, 0.51 mmol, prepared in analogy to the Bock benzodiazepine), sodium hydride, (22 mg of an 80% dispersion in oil, 0.72 mmol), and 1-bromopinacolone (179 mg, 1 mmol). The crude product was purified by flash chromatography on silica gel (eluant EtOAc:hexane fr 30:70 v/v) to afford the title compound (145 mg, 52%).
- NMR: (CDCl₃) δ 7.80 - 7.30 (12H, m); 6.80 (1H, d, J = 8Hz); 5.60 (1H, d, J = 8Hz) ; 5.30 (2H, s) ; 5.25 (1H, d, J = 17Hz); 4.60 (1H, d, J = 17Hz); 1.35 (9H, s).

### 54B

### N-((3RS)-1-tert-Butylcarbonylmethyl-2,3-dihydro-2-oxo-5-(2-chlorophenyl)-1H-1,4-benzodiazepin-3-yl-N'-(3-methylphenyl) urea (Scheme 1, step (ii))

This was prepared following the method of Reference Example 1C using the benzodiazepine of Example 54A (145 mg, 0.26 mmol), 5% palladium-on-carbon (120 mg) and m-tolyl isocyanate (40 µl, 0.32 mmol). The crude product was purified by chromatography (eluant EtOAc:hexane fr 40:60 v/v) to provide the title compound which was lyophilised from MeCN/H₂0 to give the product as a white solid (45 mg, 31%).
- NMR: (CDCl₃) δ 7.70 - 6.95 (14H, m) ; 5.80 (1H, d, J = 8 Hz) ; 5.30 (1H, d, J = 17 Hz) ; 4.60 (1H, d, J = 17 Hz) ; 2.40 (3H, s), 1.30 (9H, s).
- M.S.: (FAB, +ve ion) m/e 517.2 [M+H1
Note:
The 7-chloro substituent is lost during the hydrogenolysis.

### EXAMPLE 55

### N-((3RS)-1-Isopropylcarbonylmethyl-2,3-dihydro-2-oxo-5-phenyl-1H-1,4-benzodiazepin-3-yl-N'-(3-methylphenyl) urea. Compounds 56

### 55A

### 1-Bromo-3-methylbutan-2-one (Scheme 2, steps (i)-(iii))

This was prepared following the method of Example 3A. The intermediate diazoketone was prepared from isobutyric acid 8.82 g (100 mmol), thionyl chloride (30 ml, 400 mmol) and CH₂N₂ (generated from Diazald^{®}, 43 g, 200 mmol) and subsequently treated with a saturated solution of HBr in EtOAc. Flash chromatography on silica gel (eluant EtOAc:hexane fr 6:94 v/v) afforded the title compound as a mobile oil (300 mg, 1.8%).
- NMR: (CDCl₃) δ 4.25 (2H, s; 3.00 (1H, heptet, J = 8
Hz); 1.25 (6H, d, J = 8 Hz).

### 55B

### (3RS)-3-Benzyloxycarbonylamino-1-isopropyl-carbonylmethyl-2,3-dihydro-5-phenyl-1H-1,4-benzodiazepin-2-one (Scheme 1, step (i))

This was prepared following the method of Reference Example 1B, using the Bock benzodiazepine (606 mg, 1.5 mmol), sodium hydride (63 mg of an 80% dispersion in oil, 2.1 mmol) and the bromomethylketone of Example 55A (300 mg, 1.8 mmol). The crude product was purified by flash chromatography on silica gel (eluant EtOAc:hexane fr 40:60 v/v) to afford the title compound as a colourless crystalline solid (250 mg, 36%).
- NMR: (CDCl₃) δ 7.70 - 7.20 (14H, m); 6.75 (1H, d, J = 8 Hz); 5.50 (1H, d, J = 8Hz); 5.20 (2H, s); 4.85 (1H, d, J = 17Hz); 4.75 (1H, d, J = 17Hz); 2.80 (1H, heptet, J = 8Hz); 1.25 (6H, d, J = 8Hz).

### 55C

### N-((3RS)-1-Isopropylcarbonylmethyl-2,3-dihydro-2-oxo-5-phenyl-1H-1,4-benzodiazepin-3-yl)-N'(3-methylphenyl) urea (Scheme 1, step (ii))

This was prepared following the method of Example 27B using the benzodiazepine of Example 55B (250 mg, 0.53 mmol), BBr₃ (3.2 ml, 1.0 M sol in CH₂Cl₂) and m-tolyl isocyanate (40 µl, 0.31 mmol). The product was purified by flash chromatography on silica gel (eluant EtOAc:hexane fr 55:45 v/v) to provide the title compound which was lyophilised from MeCN/H₂0 to give the product as a white solid (51 mg, 21%, >98% pure by HPLC).
- R_{f}: (EtOAc:hexane fr 50:50) 0.16.
- NMR: (CDCl₃) δ 7.70 - 7.15 (14H, m); 6.85 (1H, d, J = 8Hz); 5.70 (1H, d, J = 8Hz); 4.75 (2H, s); 2.70 (1H, heptet, J = 8Hz); 2.30 (3H, s); 1.18 (6H, d, J = 8Hz).
- M.S.: (FAB, +ve ion) m/e 469.3 [M+H]

### EXAMPLE 56

### N-((3R)-1-tert-Butylcarbonylmethyl-2,3-dihydro-2-oxo-5-phenyl-1H-1,4-benzodiazepin-3-yl)-N'-(3-methylaminophenyl) urea Compound 57.

### 56A

### N-Methyl-3-methoxycarbonylformanilide

This was prepared following the method of Reference Example 1B, using 3-carboxyformanilide (2.28 g, 13.8mmol), sodium hydride (1.05 g of an 80% dispersion in oil, 34.5 mmol) and iodomethane (2.85 mls, 45 mmol). The crude product was purified by flash chromatography on silica gel (eluant EtOAc:hexane fr 60:40 v/v) to afford the title compound as a colourless crystalline solid (2.30 g, 86%).
- NMR: (CDCl₃) δ 8.50 (1H, s; 7.90 - 7.40 (4H, m); 3.95 (3H, s); 3.40 (3H, s).

### 56B

### N-Methyl-3-carboxyformanilide

This was prepared following the method of Reference Example 31B, using the ester of Example 56A (1.90 g, 9.8 mmol) and LiOH.H₂0 (840 mg, 20 mmol). The title compound was obtained as a colourless crystalline solid after work up (985 mg, 56%).

### 56C

### N-((3R)-1-tert-Butylcarbonylmethyl-2,3-dihydro-2-oxo-5-phenyl-1H-1,4-benzodiazepin-3-yl)-N'-(3-(N-formyl-N-methylamino)phenyl) urea (Scheme 1, step (ii))

The resolved aminobenzodiazepine of Example 26 (980 mg, 2.8 mmol) in toluene (10 ml) was treated with the isocyanate prepared from the acid of Example 56B (985 mg, 5.5 mmol) following the method of Example 38. The product was purified by flash chromatography on silica gel (eluant EtOAc:hexane fr 75:25 v/v) to provide the title compound (960 mg, 65%).
- NMR: (CDCl₃) δ 8.45 (1H, s); 7.65 - 7.05 (14H, m); 6.70 (1H, d, J = 8 Hz); 5.70 (1H, d, J = 8 Hz); 4.98 (1H, d, J = 17 Hz); 4.75 (1H, d, J = 17 Hz); 3.20 (3H, s); 1.30 (9H, s).

### 56D

### N-((3R)-1-tert-Butylcarbonylmethyl-2,3-dihydro-2-oxo-5-phenyl-1H-1,4-benzodiazepin-3-yl)-N'-(3-methylaminophenyl) urea

The benzodiazepine of Example 56C (960 mg, 1.8 mmol) was taken up in acetone (15 ml) and treated with 4N HCl (9 ml). The mixture was stirred at room temp for 3 days, then evaporated, taken up in CH₂Cl₂, washed with 5% KHCO₃, filtered (Whatman^{®} 1 PS phase separator) and evaporated. The residue was purified by flash chromatography on silica gel (eluant CHCl₃:MeOH:AcOH 120:2:1 v/v/v) to give the title compound which was crystallised from MeCN/Et₂0 to give a white solid (400 mg, 45%, >99% pure by HPLC).
- NMR: (CDCl₃) δ 7.70 - 7.20 (13H, m); 6.75 (1H, s); 6.65 (1H, d, J = 8Hz); 6.30 (1H, d, J = 8Hz); 5.75 (1H, d, J = 8Hz); 4.85 (2H, s); 2.80 (3H, s); 1.20 (9H, s).
- M.S.: (FAB +ve ion) m/e 498.3 [M+H]

### EXAMPLE 57

### N-((3RS)-1-tert-Butylcarbonylmethyl-2,3-dihydro-2-oxo-5-(2-pyridyl)-1H-1,4-benzodiazepin-3-yl)-N'-(3-methylaminophenyl) urea. Compound 58

### 57A

### N-((3RS)-1-tert-Butylcarbonylmethyl-2,3-dihydro-2-oxo-5-(2-pyridyl)-1H-1,4-benzodiazepin-3-yl)-N'-(3-(N-formyl-N-methylamino)phenyl) urea (Scheme 1, step (ii))

This was prepared following the method of Example 27B using the benzodiazepine of Example 27A (340 mg, 0.7 mmol), BBr₃ (4.4 ml, 1.0 M sol in CH₂Cl₂) and the isocyanate prepared using the acid of Example 56B (420 mg, 2.3 mmol) following the method of Example 38. The product was purified by flash chromatography on silica gel (eluant EtOAc) to provide the title compound (185 mg, 50%).
- NMR: (CDCl₃) δ 8.25 (1H, d, J = 8Hz); 8.00 (1H, s); 7.60 - 6.80 (12H, m); 6.40 (1H, d, J = 8Hz); 5.35 (1H, d, J = 8Hz); 4.62 (1H, d, J = 17Hz); 4.30 (1H, d, J = 17Hz); 2.90 (3H, s); 0.90 (9H, s).

### 57B

### N-((3RS)-1-tert-Butylcarbonylmethyl-2,3-dihydro-2-oxo-5-(2-pyridyl)-1H-1,4-benzodiazepin-N'-3-yl)-3-methylaminophenyl) urea

This was prepared following the method of Example 56D, using the benzodiazepine of Example 57A (185 mg, 0.35 mmol), acetone (5 ml) and 4N HCl (3 ml). The crude product was purified by flash chromatogaphy on silica gel (eluant CHCl₃:MeOH:AcOH 120:2:1 v/v/v) to provide the title compound which was lyophilised from MeCN/H₂0 to give the product as a white solid (67 mg, 38%, >95% pure by HPLC).
- NMR: (CDCl₃) δ 8.30 (1H, d, J = 8Hz); 7.95 (1H, d, J = 8 Hz); 7.60 (1H, t, J = 7Hz); 7.35 (1H, t, J = 7 Hz); 7.20 - 6.85 (8H, m); 6.50 (1H, d, J = 8Hz); 6.30 (1H, d, J = 8Hz); 6.10 (1H, d, J = 8Hz); 5.40 (1H, d, J = 8Hz); 4.75 (1H, d, J = 17Hz); 4.40 (1H, d, J = 17Hz); 2.60 (3H, s); 0.95 (9H, s).
- M.S.: (FAB +ve ion) m/e 499.3 [M+H]

### EXAMPLE 58

### N-((3R)-1-tert-Butylcarbonylmethyl-2,3-dihydro-2-oxo-5-phenyl-1H-1,4-benzodiazepin-3-yl)-N'-(3-(N-ethyl-N-methylamino)phenyl) urea. Compound 59

### 58A

### Methyl-3-(N-ethyl-N-methylamino)benzoate

This was prepared following the method of Example 56D using the methyl ester of Example 56A (2.35 g, 12.2 mmol), acetone (100 ml) and 4N HCl (60 ml). The resultant amine was taken up in MeOH:AcOH (99:1, 70 ml) at O°C. Acetaldehyde (1.0 ml, 18 mmol) and NaBH₃CN (1.13 g, 18 mmol) were added. The mixture was stirred at O°C for 2 hours, then evaporated, taken up in EtOAc, washed with 5% KHCO₃, brine, filtered (Whatman^{®} 1 PS phase separator) and evaporated. The residue was purified by flash chromatography on silica gel (eluant EtOAc:hexane fr 15:85 v/v) to give the title compound (2.15 g, 91%).
- NMR: (CDCl₃) δ7.40 - 7.25 (3H, m); 6.90 (1H, m); 3.90 (3H, s); 3.45 (2H, quartet, J =7Hz); 2.95 (3H, s); 1.15 (3H, t, J = 7Hz).

### 58B

### 3-(N-Ethyl-N-methylamino)benzoic acid

This was prepared following the method of Reference Example 31B, using the ester of Example 58A (2.15 g, 11.1 mmol) and LiOH.H₂0 (920 mg, 22 mmol). The crude product was purified by flash chromatography on silica gel (eluant EtOAc:hexane fr:AcOH 35:65:2) to give the title compound (1.90 g, 95%).

### 58C

### N-((3R)-1-tert-Butylcarbonylmethyl-2,3-dihydro-2-oxo-5-phenyl-1H-1,4-benzodiazepin-3-yl)-N'-(3-(N-ethyl-N-methylaminophenyl) urea (Scheme 1 step) (ii))_

The resolved aminobenzodiazepine of Example 26 (245 mg, 0.7 mmol) in toluene (5 ml) was treated with the isocyanate prepared using the acid of Example 58B (250 mg, 1.4 mmol) following the method of Example 38. The product was purified by flash chromatography on silica gel (eluant EtOAc:hexane fr 45:55 v/v) to give the title compound which was crystallised from acetonitrile to give the product as a white solid (237 mg, 64%, >99% by HPLC).
- NMR: (CDCl₃) δ 7.70 - 7.05 (13H, m); 6.58 (1H, d, J = 8Hz); 6.50 (1H, d, J =8Hz); 5.80 (1H, d, J = 8Hz); 4.90 (1H, d, J =17Hz); 4.83 (1H, d, J = 17Hz); 3.40 (2H, quartet, J =7Hz); 2.95 (3H, s); 1.25 (9H, s); 1.15 (3H, t, J = 7Hz).
- M.S.: (FAB, +ve ion) m/e 526.3 [M+H]

### EXAMPLE 59

### N-((3R)-1-tert-Butylcarbonylmethyl-2,3-dihydro-2-oxo-5-phenyl-1H-1,4-benzodiaze-pin-3-yl)-N'-(3-diethylaminophenyl) urea. Compound 60

### 59A

### 3-Ethoxycarbonyl-N-ethylformanilide

This was prepared following the method of Reference Example 1B, using 3-carboxyformanilide (5.0 g, 30 mmol), sodium hydride (2.26 g, of an 80% dispersion in oil, 75 mmol) and iodoethane (5.2 ml, 64 mmol). The product was purified by flash chromatography on silica gel (eluant EtOAc:hexane fr 50:50 v/v) to provide the title compound (6.00 g, 27.1 mmol).
- NMR: (CDCl₃) δ 8.50 (1H, s); 8.00 - 7.50 (4H, m); 4.50 (2H, q, J =7Hz); 4.00 (2H, q, J =7Hz); 1.50 (3H, t, J = 7Hz); 1.30 (3H, t, J = 7Hz).

### 59B

### Ethyl 3-diethylaminobenzoate

This was prepared using the method of Example 56D using the ethyl ester of Example 59A (3.50 g, 15.8 mmol), acetone (130 ml) and 4 N HCl (80 ml). The resultant amine was alkylated following the method of Example 58A using acetaldehyde (1.3 ml, 23.3 mmol) and NaBH₃CN (1.46 g, 23.3 mmol). The crude product was purified by flash chromatography on silica gel (eluant EtOAc:hexane fr 10:90 v/v) to give the title compound (1.90 g, 54%).
- NMR: (CDCl₃) δ 7.95 - 7.80 (3H, m); 7.40 (1H, m); 4.90 (2H, q, J =7Hz); 3.90 (4H, q, J =7Hz); 1.95 (3H, t, J = 7Hz); 1.75 (6H, t, J = 7Hz).

### 59C

### 3-Diethylaminobenzoic acid

This was prepared following the method of Reference Example 31B, using ester of Example 59B (1.90 g, 8.6 mmol) and LiOH.H₂0 (720 mg, 17.2 mmol). The crude product was purified by flash chromatography on silica gel (eluant EtOAc:hexane fr:AcOH 30:70:2 v/v/v) to give the title compound (1.0 g, 60%).

### 59D

### N-((3R)-1-tert-Butylcarbonylmethyl-2,3-dihydro-2-oxo-5-phenyl-1H-1,4-benzodiazepin-3-yl)-N'-(3-diethylaminophenyl) urea (Scheme 1, step (ii))

The resolved aminobenzodiazepine of Example 26 (175 mg, 0.5 mmol) in toluene (5 ml) was treated with the isocyanate prepared using the acid of Example 59C (240 mg, 1.2 mmol) following the method of Example 38. The product was purified by flash chromatography on silica gel (eluant EtOAc:hexane fr 45:55 v/v) to give the title compound which was crystallised from MeCN to give the product as a white solid (147 mg, 55%, >99% pure by HPLC).
- NMR: (CDCl₃) δ 7.70 - 7.05 (13H, m); 6.60 (1H, d, J = 8Hz); 6.50 (1H, d, J = 8Hz); 5.80 (1H, d, J = 8Hz); 4.90 (1H, d, J =17Hz); 4.76 (1H, d, J = 17Hz); 3.45 (4H, q, J =7Hz); 1.30 (9H, s); 1.20 (6H, t, J = 7Hz).
- M.S.: (FAB, +ve ion) m/e 540.3 [M+H]

### EXAMPLE 60

### N-((3RS)-1-tert-Butylcarbonylmethyl-2,3-dihydro-2-oxo-5-phenyl-1H-1,4-benzodiazepin-3-yl-N'-(3-dimethylaminomethylphenyl) urea. Compound 61

### 60A

### 3-Dimethylaminomethyl benzonitrile

To a solution of 3-cyanobenzaldehyde (2.50 g, 19 mmol) in MeOH:AcOH (25 ml, 99:1) was added dimethyl amine (2.5 ml, 50 mmol) at O°C, followed by NaBH₃CN (1.80 g, 29 mmol). The mixture was stirred at O°C for 18 hours, then evaporated, taken up in EtOAc, washed with 5% KHCO₃, brine filtered (Whatman^{®} 1 PS phase separator) and evaporated. The residue was purified by flash chromatography on silica gel (eluant EtOAc:MeOH 98:2 v/v) to give the title compound (1.15 g, 38 %).
- NMR: (CDCl₃) δ 7.75 - 7.60 (4H, m); 3.50 (2H, s) ; 2.35 (6H, s).

### 60B

### Methyl 3-dimethylaminomethylbenzoate

A solution of the nitrile of Example 60A, (1.15 g, 7.2 mmol) in 4M HCl/MeOH (200 ml) was stirred at O°C for 2 hours and at room temperature for 18 hours. H₂0 (25 ml) was added and the mixture was stirred for one hour at room temp, then evaporated and azeotroped with toluene, taken up in CHCl₃, washed with 5% KHCO₃, filtered (Whatman^{®} 1 PS phase separator) and evaporated to give the title compound (1.20 g, 86%).
- NMR: (CDCl₃) δ 8.00 - 7.50 (4H, m) ; 4.00 (3H, s) ; 3.60 (2H, s) ; 2.30 (6H, s).

### 60C

### 3-Dimethylaminomethylbenzoic acid

This was prepared following the method of Reference Example 31B, using the ester of Example 60B (1.20 g, 6.2 mmol) and LiOH.H₂0 (540 mg, 13 mmol) . The crude product was purified by flash chromatography on silica gel (eluant:CHCl₃:MeOH:AcOH 25:10:1 v/v/v) to give the title compound (750 mg, 68%).

### 60D

### N-((3RS)-1-tert-Butylcarbonylmethyl-2,3-dihydro-2-oxo-5-phenyl-1H-1,4-benzodiazepin-3-yl)-N'-(3-dimethylaminomethylphenyl) urea (Scheme 1. step (iii))

The benzodiazepine of Example 2B was hydrogenated as described in Reference Example 1C and the resultant amine (192 mg, 0.55 mmol) in toluene (5 ml) was treated with the isocyanate prepared using the acid of Example 60C (250 mg, 1.4 mmol) following the method of Example 38. The product was purified by flash chromatogaphy on silica gel (eluant CHCl₃:MeOH:AcOH 20:2:1 v/v/v) and crystallised from acetonitrile to give a white solid (50 mg, 17%, >99% pure by HPLC).
- NMR: (CDCl₃) δ 7.55 - 6.90 (15H, m); 5.57 (1H, d, J = 8Hz); 4.80 (1H, d, J = 17Hz); 4.70 (1H, d, J = 17Hz); 3.40 (2H, s); 2.25 (6H, s); 1.15 (9H, s).
- M.S.: (FAB, +ve ion) m/e 526.3 [M+H]

### EXAMPLE 61

### N-((3RS)-2-tert-Butylcarbonylmethyl-2,3-dihydro-2-oxo-5-(2-pyridyl)-1H-1,4-benzodiazepin-3-yl)-N'-(3-(N-ethyl-N-methylamino)phenyl) urea. Compound 62

The benzodiazepine of Example 27A was deprotected as described in Example 27B and the resultant amine (200 mg, 0.57 mmol) in toluene (5 mls) was treated with the isocyanate prepared using the acid of Example 58B (250 mg, 1.4 mmol) following the method of Example 38. The product was purified by flash chromatography on silica gel (eluant EtOAc:hexane fr 90: 10 v/v) to give the title compound which was lyophilised from MeCN/H₂O to give a white solid (125 mg, 42%, >95% pure by HPLC).
- R_{f}: (EtOAc:hexane fr 90:10) 0.23.
- M.S.: (FAB, +ve ion) m/e 527.3 [M+H]

### EXAMPLE 62

### N-((3RS)-1-tert-3-Butylcarbonylmethyl-2,3-dihydro-2-oxo-5-(2-pyridyl)-1H-1,4-benzodiazepin-3-yl)-N'-(3-dimethylaminomethylphenyl) urea. Compound 63

The benzodiazepine of Example 27A was deprotected as described in Example 27B and the resultant amine (200 mg, 0.57 mmol) in toluene (5 ml) was treated with the isocyanate prepared using the acid of Example 60C (250 mg, 1.4 mmol) following the method of Example 38. The product was purified by flash chromatogaphy on silica gel (eluant CHCl₃:MeOH:AcOH 18:4:1 v/v/v) to give the title compound which was crystallised from EtOAc-hexane fr to give a white solid (76mg, 25%, >99% pure by HPLC).
- NMR: (CDCl₃) δ 8.50 (1H, d, J = 8Hz); 8.00 - 7.20 (12H, m); 6.80 (1H, d, J = 8Hz); 5.60 (1H, d, J = 8Hz); 4.80 (1H, d, J = 17Hz); 4.50 (1H, d, J = 17Hz); 3.40 (2H, s) ; 2.20 (6H, S) ; 1.20 (9H, s).
- M.S.: (FAB, +ve ion) m/e 527.3 [M+H1

### EXAMPLE 63

### N-((3RS)-1-tert-Butylcarbonylmethyl-2,3-dihydro-2-oxo-5-(2-pyridyl)-1H-1,4-benzodiazepin-3-yl)-N'-(3-diethylaminophenyl) urea. Compound 63

The benzodiazepine of Example 27A was deprotected as described in Example 27B and the resultant amine (200 mg, 0.57 mmol) in toluene (5 ml) was treated with the isocyanate prepared using the acid of Example 59C (212 mg, 1.1 mmol) following the method of Example 38. The product was purified by flash chromatogaphy on silica gel (eluant EtOAc:hexane fr 75:25 v/v) to give the title compound which was crystallised from acetonitrile to give a white solid (200 mg, 65%, >99% pure by HPLC).
- NMR: (CDCl₃) δ 8.60 (1H, d, J = 8Hz); 8.17 (1H, d, J = 8Hz); 7.70 - 6.90 (10H, m); 6.40 (2H, m); 5.75 (H, d, J = 8Hz); 4.90 (1H, d, J = 17Hz); 4.40 (1H, d, J=17Hz); 3.30 (4H, q, J = 7Hz); 1.20 (9H, s); 1.15 (6H, t, J = 8Hz)
- M.S.: (FAB, +ve ion) m/e 541.4 [M+H]

### EXAMPLE 64

### N-((3R)-1-tert-Butylcarbonylmethyl-2,3-dihydro-2-oxo-5-pheny-1H-1,4-benzodiazepin-3-yl)N'-(3-dimethylaminophenyl) urea. Compound 65

This was prepared from the resolved aminobenzodiazepine of Example 26 (1.88 g, 5.4 mmol) following the method of Example 38. The product was purified by flash chromatography on silica gel (eluant EtOAc:hexane fr 55:45 v/v) and then lyophilised from dioxan-H₂O to give the title compound as a white solid (1.72 g, 62%, >98% pure by HPLC).
- R_{f}: (EtOAc:Hexane fr 60:40 v/v) 0.21.
- M.S.: (FAB, +ve ion) m/e 512.3 [M+H]
- [α]_{D} =: +97.3° (c = 0.776, CHCl₃)

### EXAMPLE 65

### N-((3RS)-1-tert-Butylcarbonylmethyl-2,3-dihydro-2-oxo-5-(4-methylphenyl)-1H-1,4-benzodiazepin-3-yl)-N'-(3-methylphenyl) urea. Compound 66

### 65A

### (3RS)-3-Benzyloxycarbonylamino-1-tert-butylcarbonylmethyl-2,3-dihydro-5-(4-methylphenyl)-1H-1,4-benzodiazepin-2-one (Scheme 1, step (i))

This was prepared from (3RS)-3-benzyloxycarbonylamino-2,3-dihydro-5-(4-methylphenyl)-1H-1,4-benzodiazepine-2-one (200 mg, 0.53 mmol, prepared by analogy to the Bock benzodiazepine) following the method of Example 2B. The product was purified by flash chromatography on silica gel (eluant EtOAc:hexane fr 35:65 v/v) to give the title compound as a colourless oil (252 mg, 96%).
- NMR: (CDCl₃) δ 7.53 (3H, m); 7.41 - 7.13 (9H, m); 6.68 (1H, d, J = 8Hz); 5.44 (1H, d, J = 8Hz); 5.18 (2H, s); 5.00 (1H, d, J = 17Hz); 4.67 (1H, d, J = 17Hz); 2.42 (3H, s); 1.28 (9H, s).

### 65B

### N-((3RS)-1-tert-Butylcarbonylmethyl-2,3-dihydro-2-oxo-5-(4-methylphenyl)-1H-1,4-benzodiazepin-3-yl)-N'-(3-methylphenyl) urea (Scheme 1, step (ii))

This was prepared from the benzodiazepine of Example 65A following the method of Reference Example 14B. The product was purified by flash chromatography on silica gel (eluant EtOAc:hexane fr 37:63 v/v) and lyophilised from dioxan-H₂O to give the title compound as a white solid (118 mg, 47%, >98% pure by HPLC).
- R_{f}: (ETOAc/Hexane 40:60 v/v) 0.18
- NMR: (CDCl₃) δ 9.02 - 8.92 (3H, m); 8.82 - 8.20 (11H, m); 7.10 (1H, d, J = 5Hz); 6.32 (1H, d, J = 8Hz); 6.17 (1, d, J = 8Hz); 3.82 (3H, s); 3.72 (3H, s); 2.66 (9H, s).
- M.S.: (FAB, +ve ion) m/e = 497.3 [M+H]

### EXAMPLE 66

### N-((3R)-1-tert-Butylcarbonylmethyl-2,3-dihydro-2-oxo-5-phenyl-1H-1,4-benzodiazepin-3-yl)-N'-(3-aminophenyl) urea. Compound 67

### 66A

### N-((3R)-1-tert-Butylcarbonylmethyl-2,3-dihydro-2-oxo-5-phenyl-1H-1,4-benzodiazepin3-yl)-N'-(3-nitrophenyl) urea (Scheme 1, step (ii))

The resolved aminobenzodiazepine of Example 26 was treated with m-nitrophenyl isocyanate as described in Example 45 to provide a colourless oil (490 mg, 94%).

Data identical to Example 45.

### 66B

### N-((3R)-1-tert-Butylcarbonylmethyl-3-dihydro-2-oxo-5-phenyl-1H-1,4-benzodiazepin-3-yl)-N'-(3-aminophenyl) urea

The benzodiazepine of Example 66A (245 mg, 0.468 mmol) was taken up in 1M ammonium chloride (8 ml) and treated with zinc dust (620 mg) and the mixture stirred vigorously as ethanol (8 ml) was added slowly to the mixture. After 1/2 hour the mixture was filtered and evaporated. The residue was purified by flash chromatogaphy on silica gel (eluant EtOAc:hexane fr 80:20 v/v) and lyophilised from MeCN-H₂O to give the title compound as a white solid (115 mg, 67%, >98% pure by HPLC).
- R_{f}: (EtOAc = 0.64.
- ¹H NMR: (CDCl₃) δ 7.8 - 7.0 (11H, m); 6.9 (1H, t, J = 8.5Hz); 6.75 (m, 2H); 6.35 (1H, d, J = 8.5Hz); 5.7 (1H, d, J = 8.5Hz); 4.95 (2H, s); 3.75 (2H, br.s.); 1.21 (9H, s).
- [α]_{D} =: +25° (c = 0.993, MeOH)
- M.S.: (FAB, +ve ion) ^{m}/ₑ = 484.3 [M+H]

### EXAMPLE 67

### N-((3RS)-1-tert-Butylcarbonylmethyl-7-chloro-2,3-dihydro-2-oxo-phenyl-1H-1,4-benzodiazepin-3-yl)-N'-(3-methylphenyl) urea. Compound 74

### 67A

### (3RS)-3-Benzyloxycarbonylamino-1-tert-butylcarbonylmethyl-7-chloro-2,3-dihydro-5-phenyl-1H-1,4-benzodiazepin-2-one (Scheme 1, step (i))

This was prepared from (3RS) -3-benzyloxycarbonylamino-7-chloro-2,3-dihydro-5-phenyl-1H-1,4-benzodiazepin-2-one (640 mg, 1.46 mmol, prepared by analogy with the Bock benzodiazepine), sodium hydride (62 mg of an 80% dispersion in oil, 2.04 mmol) and 1-bromopinacolone (537 mg, 3 mmol) following the method of Reference Example 1B. The crude product was purified by flash chromatography on silica gel (eluant EtOAc:hexane fr 30:70 v/v) to afford the title compound (700 mg, 93%).
- NMR: (CDCl₃) δ 7.70 - 7.30 (13H, m); 6.8 (1H, d, J = 8Hz); 5.60 (1H, d, J = 8Hz); 5.30(2H, s); 5.05 (1H,d, J = 17Hz); 4.80 (1H, d, J = 17Hz); 1.35 (9H, s).

### 67B

### N-((3RS)-1-tert-Butylcarbonylmethyl-7-chloro-2,3-dihydro-2-oxo-5-phenyl-1H-1,4-benzodiazepin-3-yl)-N'-(3-methylphenyl) urea (Scheme 1, step (ii))

This was prepared following the method of Example 27B using the benzodiazepine of Example 67A (270 mg, 0.52 mmol), BBr₃ (3.4 ml, 1.0 M sol in CH₂Cl₂) and m-tolyl isocyanate (90 µl, 0.66 mmol). The product was purified by chromatography (eluant EtOAc:hexane fr 40:60 v/v) to provide the title compound which was freeze dried from MeCN/H₂O to give the product as a white solid (83 mg, 31%, >99% by HPLC).
- NMR: (CDCl₃) δ 7.70 - 6.95 (14H, m); 5.80 (1H, d, J = 8Hz); 4.90 (2H, s); 2.40 (3H, s); 1.25 (9H, s).
- M.S.: (FAB, +ve ion) m/e 517.2 [M+H]

### EXAMPLE 68

### N-((3RS)-1-tert-Butylcarbonylmethyl-7-chloro-2,3-dihydro-2-oxo-5-phenyl-1H-1,4-benzodiazepin-3-yl)-N'-(3-dimethylaminophenyl) urea. Compound 75

This was prepared following the method of Example 27B using the benzodiazepine of Example 67A (430 mg, 0.83 mmol), BBr₃ (5.0 ml, 1.0 M sol in CH₂Cl₂) and the isocyanate prepared using 3-dimethylaminobenzoic acid (400 mg, 2.4 mmol) following the method of Example 38. The product was purified by flash chromatography on silica gel (eluant EtOAc:hexane fr 45:55 v/v) to provide the title compound which was lyophilised from MeCN/H₂O to give a white solid (100 mg, 22%, >98% pure by HPLC).
- NMR: (CDCl₃) δ 7.60 - 6.90 (12H, m); 6.50 (1H, d, J = 8Hz); 6.40 (1H, d, J = 8Hz); 5.60 (1H, d, J = 8Hz); 4.75 (2H, s); 2.90 (6H, s); 2.10 (3H, s); 1.15 (9H, s).
- M.S.: (FAB, +ve ion) m/e 546.3 [M+H]

### EXAMPLE 69

### N-((3RS)-1-tert-Butylcarbonylmethyl)-7-chloro-2,3-dihydro-2-oxo-5-(2-chlorophenyl)-1H-1,4-benzodiazepin-3-yl)-N'-(3-methylphenyl) urea. Compound 76

This was prepared following the method of Example 27B using the benzodiazepine of Example 54A (170 mg, 0.31 mmol), BBr₃ (2 ml, 1.0 M sol in CH₂Cl₂) and m-tolyl isocyanate (60 µl, 0.4 mmol). The product was purified by flash chromatography on silica gel (eluant EtOAc:hexane fr 35:65 v/v) to provide the title compound which was lyophilised from MeCN/H₂O to give a white solid (39 mg, 5 1 %, >99% pure by HPLC).
- R_{f}: (EtOAc:hexane fr 40:60) 0.25.
- M.S.: (FAB, +ve ion) m/e 551.2 [M+H]

### EXAMPLE 70

### N-((3RS)-1-tert-Butylcarbonylmethyl-2,3-dihydro-8-methyl-2-oxo-5-phenyl-1H-1,4-benzodiazepin-3-yl)-N'-(3-methylphenyl) urea. Compound 77

### 70A

### (3RS)-3-Benzyloxycarbonylamino-tert-1-butylcarbonylamino-2,3-dihydro-8-methyl-5-phenyl-1H-1,4-benzodiazepin-2-one (Scheme 1, step (i))

This was prepared from (3RS)-3-benzyloxycarbonylamino-2,3-dihydro-8-methyl-5-phenyl-1H-1,4-benzodiazepin-2-one (240 mg, 0.64 mmol, prepared by analogy to the Bock benzodiazepine) following the method of Example 2B. The product was purified by flash chromatography on silica gel (eluant EtOAc:hexane fr 30:70 v/v) to give the title compound as a colourless oil (289 mg, 91%).
- R_{f}: (EtOAc:hexane fr 30:70 v/v) 0.17.
- NMR: (CDCl₃) δ 7.67 (2H, m); 7.4 (9H, m); 7.25 (1H, d, J = 8Hz); 6.96 (1H, s); 6.70 (1H, d, J = 9Hz); 5.47 (1H, d, J = 9Hz); 5.19 (2H, s); 4.98 (1H, d, J=18Hz); 4.70 (1H, d, J = 18Hz); 2.46 (3H, s); 1.30 (9H, s).

### 70B

### N-((3RS)-1-tert-Butylcarbonylmethyl-2,3-dihydro-8-methyl-2-oxo-5-phenyl-1H-1,4-benzodiazepin-3-yl)-N'-(3-methylphenyl) urea (Scheme 1, step (ii))

This was prepared from the benzodiazepine of Example 70A (289 mg, 0.58 mmol) following the method of Reference Example 14B. The product was purified by flash chromatography on silica gel (eluant EtOAc:hexane fr 37:63 v/v) and lyophilised from dioxan-H₂O to give the title compound as a white solid (106 mg, 37%, >98% pure by HPLC).
- R_{f}: (EtOAc:hexane fr 40:60 V/V) 0.18.
- NMR: (CDCl₃) δ 7. 67 (2H, m); 7.46 (2H, m); 7.26 - 6.79 (9H, m); 5.68 (1H, d, J = 7Hz); 4.93 (1H, d, J = 11Hz); 4.75 (1H, d, J = 11Hz); 2.45 (3H, s); 2.33 (3H, s); 1.27 (9H, s).
- M.S.: (FAB, +ve ion) m/e = 497.2 [M+H]

Compounds 53, 68-73, 78 and 79 are obtainable by methods analogous to those described in the above Examples and Reference Examples.

The compounds of the present invention are potent and selective antagonists at the CCK-B receptor and inhibit gastric acid secretion stimulated by pentagastrin. The methods of measuring these activities are described below:

### Measurement of binding affinity for CCK-B receptors

About 100 SD rats were decapitated without anaesthesia, the whole brain was immediately excised from each of the rats and homogenized in 10-fold volume of 0.32 M aqueous solution of sucrose by the use of a Teflon-coated homogenizer, the homogenate thus obtained was centrifuged for ten minutes at 900 g by the use of a cooled centrifuge, and the supernatant was further centrifuged for 15 minutes at 11500 g. The precipitate thus obtained was dispersed in 50 mM Tris-HCl buffer (pH 7.4) containing 0.08% Triton X-100, this suspension was allowed to stand for 30 minutes and again centrifuged for 15 minutes at 11500 g, the precipitate thus obtained was washed twice with 5 mM Tris-HCl buffer and twice with 50 mM Tris-HCl buffer in that order with centrifugal separation, the washed precipitate was suspended in 50 mM Tris-HCl buffer, and the suspension thus obtained was stored at -80°C until the membrane preparation was required.

The membrane preparations were warmed to room temperature, diluted with 10 mM HEPES buffer (containing 130 mM NaCl, 5 mM MgCl₂, 1 mM EGTA and 0.25 mg/ml bacitracin; pH 6.5) and incubated at 25°C for 120 minutes in the presence of [¹²⁵I]BH-CCK-8 and the test compound, then separated by suction filtration. Non-specific binding was determined in the presence of 1 µM CCK-8. The amount of labelled ligand bound to the receptor was measured by the use of a γ-counter; IC₅₀ values were determined, being that concentration of test compound required to inhibit specific binding by 50%.

### Measurement of binding affinity for CCK-A receptors

The pancreas of an SD rat was homogenized in a 20-fold volume of 50 mM Tris-HCl buffer (pH 7.7) by the use of a Polytrone-type homogenizer, the homogenate was twice centrifuged for 10 minutes at 50000 g by the use of an ultra-centrifuge, the precipitate thus obtained was suspended in a 40-fold volume of 50 mM Tris-HCl buffer (containing 0.2% BSA, 5 mM MgCl₂, 0.1 mg/ml bacitracin and 5 mM DTT; pH 7.7), and the suspension was stored at -80°C until the membrane preparations were required.

The membrane preparations were then warmed to room temperature, diluted 1:10 with the buffer and incubated at 37°C for 30 minutes in the presence of [³H]L-364,718 and the test compound then separated by suction filtration. Non-specific binding was determined in the presence of 1 µM L-364,718. The amount of labelled ligand bound to the receptor was measured by the use of a liquid scintillation counter; IC₅₀ values were determined, being that concentration of test compound required to inhibit specific binding by 50%

A high affinity for the CCK-A receptor in a gastrin/CCK-B antagonist is thought to be undesirable as it may lead to side-effects such as cholestasis and gall stone formation during therapy. Therefore it is preferable for the therapeutic agent to be selective for the CCK-B receptor. This seletivity is expressed by the ratio IC₅₀ (CCK-A)/IC₅₀ (CCK-B); the higher the value of this ratio the better is the selectivity.

The table below summarises CCK-B and CCK-A binding data for examples of preferred compounds, as well as the A/B ratio. Many compounds display a marked increase in CCK-B receptor binding affinity when compared to the compound of Example 281 of U.S. Patent No. 4,820,834 (also known as L-365,260). Several compounds also show much greater selectivity for the CCK-B receptor over the CCK-A receptor than that reported for the compound of Example 281 of U.S. Patent No.4,820,834.

| **Receptor binding affinity IC₅₀(nM)** | | | |
|---|---|---|---|
| **COMPOUND** | **CCK-B** | **CCK-A** | **A/B Ratio** |
| Compound of Example 281 of US Patent No. 4,820,834 | 29 | 12,000 | 410 |
| | | | |
| Example 36 | 0.57 | 720 | 1,300 |
| Example 37 | 1.1 | 5,600 | 5,100 |
| Example 39 | 0.10 | 240 | 2,400 |
| Example 41 | 6.2 | 6,700 | 1,100 |
| Example 45 | 0.16 | 590 | 3,700 |
| Example 56 | 0.11 | 120 | 1,100 |
| Example 66 | 0.5 | 950 | 1,900 |

### Measurement of inhibition of pentagastrin-stimulated gastric acid secretion in dog

Assays were performed using male beagle dogs (7 - 12 kg) two months after surgical preparation of Heidenhain pouches following the conventional procedure. Each dog was used only once per week.

The animals were deprived of food with free access to water for 18 hr prior to the experiment. The gastric juice was collected from the gastric canula by gravity drainage every 15 min, and acid output was determined by automatic titration with 0.05 N NaOH to pH 7.0 (Comtite-7, Hiranuma, Tokyo, Japan). Drugs were administered orally 3 hr after the start of the pentagastrin (8 µg/kg/hr) infusion. The effect of each drug was calculated as the percentage inhibition of stimulated acid output. The table below shows the maximum inhibition observed for representative Examples. Each result is a mean for 3 - 5 animals.

| | Inhibidon (dose) |
|---|---|
| Compound of Example 281 of US Patent No. 4,820,834 | 0% (100 µmol/kg) |
| Compound of Example 26 | 53% (3 µmol/kg) |
| Compound of Example 27 | 66% (3 µmol/kg) |
| Compound of Example 38 | 100% (3 µmol/kg) |
| Compound of Example 64 | 100% (3 µmol/kg) |

The experiments described above demonstrate that the compounds of the present invention are potent selective gastrin/CCK-B antagonists and inhibit the stimulation of gastric acid release due to pentagastrin. They are therefore useful in the treatment of disease states in which gastrin or CCK-B receptor is implicated as a mediating factor. Such disease states would include disorders of the gastro-intestinal system, for example gastric and duodenal ulcers, gastritis, reflux esophagitis, Zollinger-Ellison syndrome, gastrin-sensitive pancreas, and gastrin-sensitive tumors. Disorders of the central nervous system such as anxiety and psychoses would also be amenable to treatment with the compounds of this invention. The compounds can also be used in the control of appetite and pain.

The compounds of this invention and salts thereof can be administered orally (including sublingual administration) or parenterally in the form of tablets, powders, capsules, pills, liquids, injections, suppositories, ointments and adhesive plasters.

The carrier and excipient for pharmaceutical manufacturing can be a solid or liquid, non-toxic medicinal substance, such as lactose, magnesium stearate, starch, talc, gelatin, agar, pectin, gum arabic, olive oil, sesame oil, cocoa butter, ethylene glycol and other commonly employed materials.

Examples of formulations using the compounds of this invention are described below.

### Examples of tablet preparation

| Composition | 20 mg-Tablet | 40 mg-Tablet |
|---|---|---|
| Compound of Reference Example 4 | 20 mg | 40 mg |
| Lactose | 73.4 | 80 |
| Corn Starch | 18 | 20 |
| Hydroxypropylcellulose | 4 | 5 |
| Carboxymethylcellulose Ca | 4 | 4.2 |
| Mg Stearate | 0.6 | 0.8 |
| Total | 120 mg | 150 mg |

### Preparation of 20 mg-tablets

Compound of Reference Example 4 (100 g), lactose (367 g) and corn starch (90 g) were homogeneously mixed together by the use of a flow granulating coater (product of Ohgawara Seisakusho), 10% aqueous solution of hydroxypropylcellulose (200 g) was sprayed into the mixture, and granulation was then performed. After drying, the granules were filtered through a 20-mesh sieve, 20 g of carboxymethylcellulose Ca and 3 g of magnesium stearate were then added, and the mixture was treated in a rotary tablet machine equipped with a pestle of 7 mm x 8.4 R (product of Hata Tekkosho), thus producing tablets each weighing 120 mg

### Preparation of 40 mg-tablets

Compound of Example 4 (140 g), lactose (280 g) and corn starch (70 g) were homogeneously mixed together by the use of a flow granulating coater (product of Ohgawara Seisakusho), 10% aqueous solution of hydroxypropylcellulose (175 g) was sprayed into the mixture, and granulation was then performed. After drying, the granules were filtered through a 20-mesh sieve, 14.7 g of carboxymethylcellulose Ca and 2.8 g of magnesium stearate were then added, and the mixture was treated in a rotary tablet machine equipped with a pestle of 7.5 mm x 9R (product of Hata Tekkosho), thus producing tablets each weighing 150 mg.

The clinical dosage of the compounds of this invention will be determined by the physician taking into account the precise illness, and the body weight, age, sex, medical history and other factors of the patient to be treated. In general the dosage when administered orally will be between 1 and 1000 mg/day in either a single dose or sub-divided into smaller multiple doses.

## Claims

1. A benzodiazepine derivative of formula I or a pharmaceutically acceptable salt thereof: 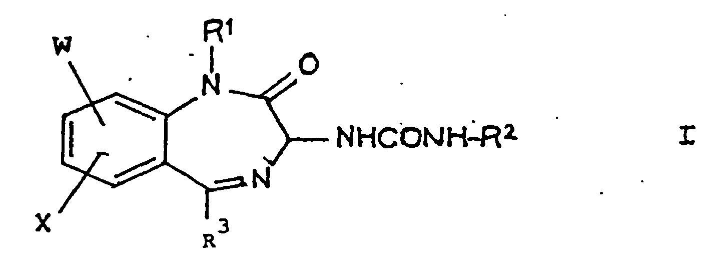 wherein
(a) R¹ is -CH₂CHOH(CH₂)ₐR⁴ or a ketone group -CH₂CO(CH₂)ₐR⁵ in which a is 0 or 1 and R⁴ and R⁵ are selected from alkyl groups of up to 8 carbon atoms;
(b) R² and R³ are independently selected from optionally substituted phenyl and pyridyl, the optional substituents being selected from halogen atoms and hydroxy, amino, nitro, carboxylic acid, carboxamido and cyano groups and alkyl, alkoxy, alkylamino and dialkylamino groups in which the or each alkyl group is of up to 8 carbon atoms; and
(c) W and X are selected independently from halogen and hydrogen atoms and C₁-C₈ alkyl and C₁-C₈ alkoxy groups.

2. A compound according to claim 1 wherein R⁴ is C₄-C₇ linear or branched alkyl or R⁵ is C₁-C₃ alkyl or as defined for R⁴.

3. A compound according to claim 1 or 2 wherein at least one of R² and R³ is unsubstituted or substituted phenyl or pyridyl.

4. A compound according to claim 3 wherein at least one of R² and R³ is unsubstituted, monosubstituted or disubstituted phenyl or unsubstituted, monosubstituted or disubstituted 2-, 3- or 4-pyridyl.

5. A compound according to any preceding claim wherein
R¹ is -CH₂CO(CH₂)ₐR⁵,
R² is unsubstituted phenyl; phenyl having a meta substituent chosen from F, Cl, Br, OH, OCH₃, NH₂, NMe2, NO₂, Me, (CH₂)_{c}-CO₂H, CN, NHMe, MMeEt, NEt₂, CH₂NMe₂, NHCHO and (CH₂)_{c}-SO₃H where c is 0-2; or 2-, 3- or 4-pyridyl optionally with a substituent selected from F, Cl, CH₃ and CO₂H; and
R³ is phenyl or 2-, 3- or 4-pyridyl.

6. A compound according to any of claims 1 to 4 wherein R¹ is -CH₂CHOH(CH₂)ₐR⁴ and R² and R³ are as defined in claim 5.

7. A compound according to any preceding claim wherein the absolute configuration at the 3-position of the benzodiazepine ring is R (as shown in IV)

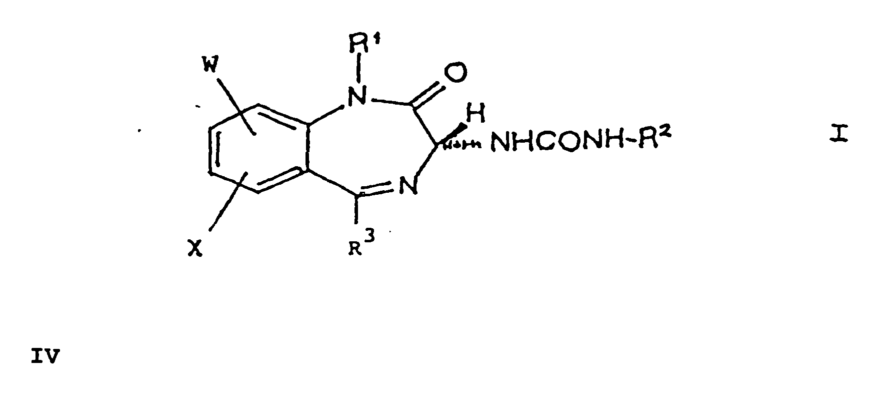

8. A compound according to any preceding claim wherein W and X are H.

9. At least one compound selected from the following compounds according to claim 1 and pharmaceutically acceptable salts thereof:
N-((3RS)-1-*tert*-Butylcarbonylmethyl-2,3-dihydro-2-oxo-5-phenyl-1H-1,4-benzodiazepin-3-yl)-N'-(3-methylphenyl) urea (Example 2);
N-((3RS)-1-Diethylmethylcarbonylmethyl-2,3-dihydro-2-oxo-5-phenyl-1H-1,4-benzodiazepin-3-yl)-N'-(3-methylphenyl) urea (Example 3);
N-((3RS)-1-*tert*-Butylcarbonylmethyl-2,3-dihydro-2-oxo-5-phenyl-1H-1,4-benzodiazepin-3-yl)-N'-(3-cyanophenyl) urea
N-((3R)-1-*tert*-Butylcarbonylmethyl-2,3-dihydro-2-oxo-5-phenyl-1H-1,4-benzodiazepin-3-yl)-N'-(3-methylphenyl) urea (Example 26);
N-((3RS)-1-*tert*-Butylcarbonylmethyl-2,3-dihydro-2-oxo-5-(2-pyridyl)-1H-1,4-benzodiazepin-3-yl)-N'-(3-methylphenyl) urea (Example 27);
N-((3RS)-1-*tert*-Butylcarbonylmethyl-2,3-dihydro-2-oxo-5-phenyl-1H-1,4-benzodiazepin-3-yl)-N'-(3-carboxyphenyl) urea (Example 28);
N-((3RS)-1-*tert*-Amylcarbonylmethyl-2,3-dihydro-2-oxo-5-phenyl-1H-1,4-benzodiazepin-3-yl)-N'-(3-methylphenyl) urea (Example 36);
N-((3RS)-1-*tert*-Amylcarbonylmethyl-2,3-dihydro-2-oxo-5-phenyl-1H-1,4-benzodiazepin-3-yl)-N'-(3-carboxyphenyl) urea (Example 37);
N-((3RS)-1-*tert*-Butylcarbonylmethyl-2,3-dihydro-2-oxo-5-(2-pyridyl)-1H-1,4-benzodiazepin-3-yl)-N'-(3-dimethylaminophenyl) urea (Example 38);
N-((3RS)-1-*tert*-Butylcarbonylmethyl-2,3-dihydro-2-oxo-5-(2-pyridyl)-1H-1,4-benzodiazepin-3-yl)-N'-(3-carboxyphenyl) urea (Example 39);
N-((3RS)-1-*tert*-Butylcarbonylmethyl-2,3-dihydro-2-oxo-5-phenyl-1H-1,4-benzodiazepin-3-yl)-N'-(3-dimethylaminophenyl) urea (Example 41);
N-((3RS)-1-*tert*-Butylcarbonylmethyl-2,3-dihydro-2-oxo-5-phenyl-1H-1,4-benzodiazepin-3-yl)-N'-(3-methoxyphenyl) urea (Example 43);
N-((3RS)-1-*tert*-Butylcarbonylmethyl-2,3-dihydro-2-oxo-5-phenyl-1H-1,4-benzodiazepin-3-yl)-N'-(3-nitrophenyl) urea (Example 45);
N-((3RS)-1-*tert*-Butylcarbonylmethyl-2,3-dihydro-2-oxo-5-phenyl-1H-1,4-benzodiazepin-3-yl)-N'-(3-formylaminophenyl) urea (Example 47);
N-((3R)-1-((2R)-2-Hydroxy-3,3-dimethylbutyl)-2,3-dihydro-2-oxo-5-phenyl-1H-1,4-benzodiazepin-3-yl)-N'-(3-methylphenyl) urea (Example 48);
N-((3R)-1-((2S)-2-Hydroxy-3,3-dimethylbutyl)-2,3-dihydro-2-oxo-5-phenyl-1H-1,4-benzodiazepin-3-yl)-N'-(3-methylphenyl)urea;
N-((3RS)-1-*tert*-Butylcarbonylmethyl-2,3-dihydro-2-oxo-5-(2-chlorophenyl)-1H-1,4-benzodiazepin-3-yl)-N'-(3-methylphenyl) urea (Example 54);
N-((3RS)-1-Isopropylcarbonylmethyl-2,3-dihydro-2-oxo-5-phenyl-1H-1,4-benzodiazepin-3-yl)-N'-(3-methylphenyl) urea (Example 55);
N-((3R)-1-*tert*-Butylcarbonylmethyl-2,3-dihydro-2-oxo-5-phenyl-1H-1,4-benzodiazepin-3-yl)-N'-(3-methylaminophenyl) urea (Example 56);
N-((3RS)-1-*tert*-Butylcarbonylmethyl-2,3-dihydro-2-oxo-5-(2-pyridyl)-1H-1,4-benzodiazepin-3-yl)-N'-(3-methylaminophenyl) urea (Example 57);
N-((3R)-1-*tert*-Butylcarbonylmethyl-2,3-dihydro-2-oxo-5-phenyl-1H-1,4-benzodiazepin-3-yl)-N'-(3-(N-ethyl-N-methylamino)phenyl) urea (Example 58);
N-((3R)-1-*tert*-Butylcarbonylmethyl-2,3-dihydro-2-oxo-5-phenyl-1H-1,4-benzodiazepin-3-yl)-N'-(3-diethylaminophenyl) urea (Example 59);
N-((3RS)-1-*tert*-Butylcarbonylmethyl-2,3-dihydro-2-oxo-5-phenyl-1H-1,4-benzodiazepin-3-yl)-N'-(3-dimethylaminomethylphenyl) urea (Example 60);
N-((3RS)-1-*tert*-Butylcarbonylmethyl-2,3-dihydro-2-oxo-5-(2-pyridyl)-1H-1,4-benzodiazepin-3-yl)-N'-(3-(N-ethyl-N-methylamino)phenyl) urea (Example 61);
N-((3RS)-1-*tert*-Butylcarbonylmethyl-2,3-dihydro-2-oxo-5-(2-pyridyl)-1H-1,4-benzodiazepin-3-yl)-N'-(3-dimethylaminomethylphenyl) urea (Example 62);
N-((3RS)-1-*tert*-Butylcarbonylmethyl-2,3-dihydro-2-oxo-5-(2-pyridyl)-1H-1,4-benzodiazepin-3-yl-N'-(3-diethylaminophenyl urea (Example 63);
N-((3R)-1-*tert*-Butylcarbonylmethyl-2,3-dihydro-2-oxo-5-phenyl-1H-1,4-benzodiazepin-3-yl)-N'-(3-dimethylaminophenyl) urea (Example 64);
N-((3RS)-1-*tert*-Butylcarbonylmethyl-2,3-dihydro-2-oxo-5-(4-methylphenyl)-1H-1,4-benzodiazepin-3-yl)-N'-(3-methylphenyl) urea (Example 65);
N-((3R)-1-*tert*-Butylcarbonylmethyl-2,3-dihydro-2-oxo-5-phenyl-1H-1,4-benzodiazepin-3-yl)-N'-(3-aminophenyl) urea (Example 66);
N-((3R)-1-*tert*-Butylcarbonylmethyl-2,3-dihydro-2-oxo-5-(2-pyridyl)-1H-1,4-benzodazepin-3-yl)-N'-(3-methylphenyl) urea;
N-((3R)-1-*tert*-Butylcarbonylmethyl-2,3-dihydro-2-oxo-5-(2-pyridyl)-1H-1,4-benzodiazepin-3-yl)-N'-(3-aminophenyl) urea;
N-((3R)-1-*tert*-Butylcarbonylmethyl-2,3-dihydro-2-oxo-5-(2-pyridyl)-1H-1,4-benzodiazepin-3-yl)-N'-(3-methylaminophenyl) urea;
N-((3R)-1-*tert*-Butylcarbonylmethyl-2,3-dihydro-2-oxo-5-(2-pyridyl)-1H-1,4-benzodiazepin-3-yl)-N'-(3-dimethylaminophenyl) urea;
N-((3RS)-1-*tert*-Butylcarbonylmethyl-2,3-dihydro-2-oxo-5-phenyl-1H-1,4-benzodiazepin-3-yl)-N'-(3-aminophenyl) urea;
N-((3RS)-1-*tert*-Butylcarbonylmethyl-2,3-dihydro-2-oxo-5-phenyl-1H-1,4-benzodiazepin-3-yl)-N'-(3-methylaminophenyl) urea;
N-((3RS)-1-*tert*-Butylcarbonylmethyl-7-chloro-2,3-dihydro-2-oxo-5-phenyl-1H-1,4-benzodiazepin-3-yl)-N'-(3-methylphenyl) urea (Example 67);
N-((3RS)-1-*tert*-Butylcarbonylmethyl-7-chloro-2,3-dihydro-2-oxo-5-phenyl-1H-1,4-benzodiazepin-3-yl)-N'-(3-dimethylaminophenyl) urea (Example 68);
N-((3RS)-1-*tert*-Butylcarbonylmethyl)-7-chloro-2,3-dihydro-2-oxo-5-(2-chlorophenyl)-1H-1,4-benzodiazepin-3-yl)-N'-(3-methylphenyl) urea (Example 69);
N-((3RS)-1-*tert*-Butylcarbonylmethyl-2,3-dihydro-8-methyl-2-oxo-5-phenyl-1H-1,4-benzodiazepin-3-yl)-N'-(3-methylphenyl) urea (Example 70);
N-((3RS)-1-*tert*-Butylcarbonylmethyl-2,3-dihydro-2-oxo-5-phenyl-1H-1,4-benzodiazepin-3-yl)-N'-(3-(N-ethyl-N-methylaminophenyl) urea;
N-((3RS)-1-*tert*-Butylcarbonylmethyl-2,3-dihydro-2-oxo-5-phenyl-1H-1,4-benzodiazepin-3-yl)-N'-(3-diethylaminophenyl) urea.

10. A medicinal composition containing as an active ingredient a compound according to any preceding claim.

11. A medicinal composition according to claim 10 which acts as a gastrin or CCK-B receptor antagonist.

12. A medicinal composition according to claim 11 which is a drug for the treatment of diseases induced by the failure of a physiological function controlled by gastrin, such as gastric and duodenal ulcers, gastritis, reflux esophagitis, gastric and colon cancers, and Zollinger-Ellison syndrome.

13. A medicinal composition according to claim 11 which is a drug for the treatment of diseases induced by the failure of physiological function controlled by the central CCK-B receptor (e.g. for the reduction of anxiety or for appetite regulation).

14. A process for the production of benzodiazepine of general formula I or pharmaceutically active salt thereof (wherein R¹ is -CH₂CO(CH₂)ₐR⁵ and a, R² , R³, R⁵, W, and X are as defined in claim 1), which comprises the reaction of 3-aminobenzodiazepine V with organic isocyanate R²-NCO:

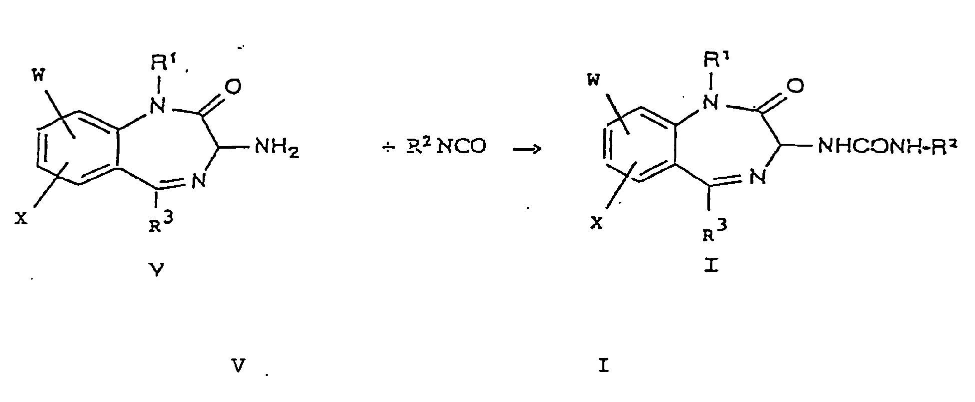

15. A process for the production of benzodiazepine of general formula I or pharmaceutically acceptable salt thereof (wherein R¹ is -CH₂CO(CH)₂ₐR⁵ and a, R², R³, R⁵, W, and X are as defined in claim 1), which comprises the reaction of 3-p-nitrophenyloxycarbonylamino benzodiazepine VI with amine R₂-NH₂ :

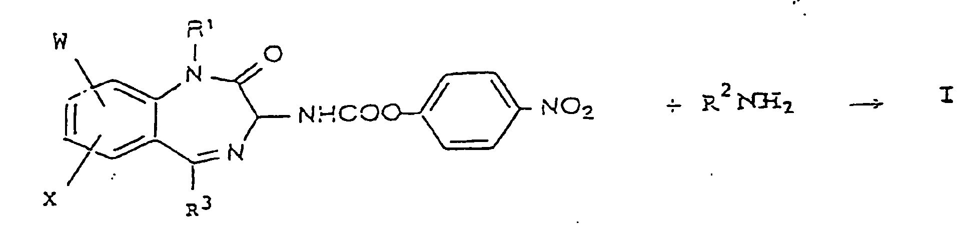

16. A process for the production of benzodiazepine of general formula I or pharmaceutically active salt thereof (wherein R¹ is -CH₂CHOH(CH₂)ₐR⁴ and a, R², R³, R⁴, W, and X are as defined in claim 1), which comprises the reaction of I with reducing agent.

17. The use of a compound according to any of claims 1 to 9 for the preparation of a medicament for the prevention or treatment of disease induced by failure of physiological function controlled by gastrin or central CCK-B receptor.

## Patentansprüche

1. Benzodiazepin-Derivat der Formel I oder ein pharmazeutisch akzeptables Salz davon: 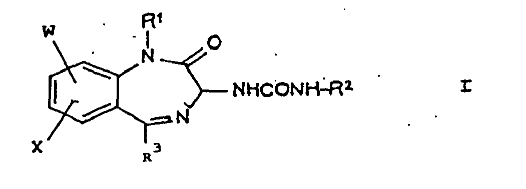 wobei
(a) R¹ -CH₂CHOH(CH₂)ₐR⁴ oder eine Ketongruppe -CH₂CO(CH₂)ₐR⁵ ist, wobei a 0 oder 1 ist und R⁴ und R⁵ ausgewählt sind aus Alkylgruppen mit bis zu 8 Kohlenstoffatomen;
(b) R² und R³ unabhängig ausgewählt sind aus optional substituiertem Phenyl und Pyridyl, wobei die optionalen Substituenten ausgewählt sind aus Halogenatomen und Hydroxyl-, Amino-, Nitro-, Carbonsäure-, Carboxamido- und Cyanogruppen und Alkyl-, Alkoxy-, Alkylamino- und Dialkylaminogruppen, wobei die oder jede Alkylgruppe aus bis zu 8 Kohlenstoffatomen besteht; und
(c) W und X unabhängig ausgewählt sind aus Halogen- und Wasserstoffatomen und C₁-C₈-Alkyl- und C₁-C₈-Alkoxygruppen.

2. Verbindung nach Anspruch 1, wobei R⁴ lineares oder verzweigtes C₄-C₇-Alkyl ist oder R⁵ C₁-C₃-Alkyl ist oder der Definition für R⁴ entspricht.

3. Verbindung nach Anspruch 1 oder 2, wobei R² und/oder R³ unsubstituiertes oder substituiertes Phenyl oder Pyridyl ist/sind.

4. Verbindung nach Anspruch 3, wobei R² und/oder R³ unsubstituiertes, monosubstituiertes oder disubstituiertes Phenyl oder unsubstituiertes, monosubstituiertes oder disubstituiertes 2-, 3- oder 4-Pyridyl ist/sind.

5. Verbindung nach einem der vorherigen Ansprüche, wobei
R¹ -CH₂CO(CH₂)ₐR⁵ ist,
R² unsubstituiertes Phenyl ist; wobei Phenyl einen meta-Substituenten hat, ausgewählt aus F, Cl, Br, OH, OCH₃, NH₂, NMe2, NO₂, Me, (CH₂)_{c}-CO₂H, CN, NHMe, MMeEt, NEt₂, CH₂NMe₂, NHCHO und (CH₂)_{c}-SO₃H, wobei c 0-2 ist; oder 2-, 3- oder 4-Pyridyl, optional mit einem Substituenten, ausgewählt aus F, Cl, CH₃ und CO₂H; und
R³ Phenyl oder 2-, 3- oder 4-Pyriyl ist.

6. Verbindung nach einem der Ansprüche 1 bis 4, wobei R¹ - CH₂CHOH(CH₂)ₐR⁴ ist und R² und R³ der Definition in Anspruch 5 entsprechen.

7. Verbindung nach einem der vorherigen Ansprüche, wobei die absolute Konfiguration in der 3-Position des Benzodiazepinrings R ist (wie in IV dargestellt)

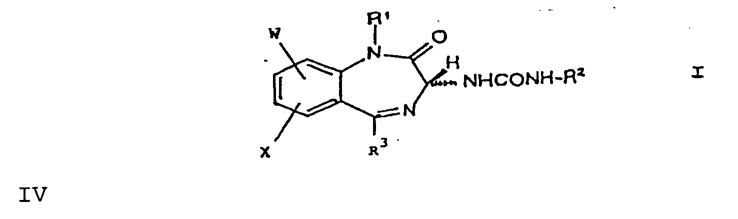

8. Verbindung nach einem der vorherigen Ansprüche, wobei W und X H sind.

9. Wenigstens eine Verbindung, ausgewählt aus den folgenden Verbindungen nach Anspruch 1, und pharmazeutisch akzeptable Salze davon:
N-((3RS)-1-*tert*-Butylcarbonylmethyl-2,3-dihydro-2-oxo-5-phenyl-1H-1,4-benzodiazepin-3-yl)-N'-(3-methylphenyl)harnstoff (Beispiel 2);
N-((3RS)-1-Diethylmethylcarbonylmethyl-2,3-dihydro-2-oxo-5-phenyl-1H-1,4-benzodiazepin-3-yl)-N'-(3-methylphenyl)harnstoff (Beispiel 3);
N-((3RS)-1-*tert*-Butylcarbonylmethyl-2,3-dihydro-2-oxo-5-phenyl-1H-1,4-benzodiazepin-3-yl)-N'-(3-cyanophenyl)harnstoff;
N-((3R)-1-*tert*-Butylcarbonylmethyl-2,3-dihydro-2-oxo-5-phenyl-1H-1,4-benzodiazepin-3-yl)-N'-(3-methylphenyl)harnstoff (Beispiel 26);
N-((3RS)-1-*tert*-Butylcarbonylmethyl-2,3-dihydro-2-oxo-5-(2-pyridyl)-1H-1,4-benzodiazepin-3-yl)-N'-(3-methylphenyl)harnstoff (Beispiel 27);
N-((3RS)-1-*tert*-Butylcarbonylmethyl-2,3-dihydro-2-oxo-5-phenyl-1H-1,4-benzodiazepin-3-yl)-N'-(3-carboxyphenyl)harnstoff (Beispiel 28);
N-((3RS)-1-*tert*-Amylcarbonylmethyl-2,3-dihydro-2-oxo-5-phenyl-1H-1,4-benzodiazepin-3-yl)-N'-(3-methylphenyl)harnstoff (Beispiel 36);
N-((3RS)-1-*tert*-Amylcarbonylmethyl-2,3-dihydro-2-oxo-5-phenyl-1H-1,4-benzodiazepin-3-yl)-N'-(3-carboxyphenyl)harnstoff (Beispiel 37);
N-((3RS)-1-*tert*-Butylcarbonylmethyl-2,3-dihydro-2-oxo-5-(2-pyridyl)-1H-1,4-benzodiazepin-3-yl)-N'-(3-dimethylaminophenyl)harnstoff (Beispiel 38);
N-((3RS)-1-*tert*-Butylcarbonylmethyl-2,3-dihydro-2-oxo-5-(2-pyridyl)-1H-1,4-benzodiazepin-3-yl)-N'-(3-carboxyphenyl)harnstoff (Beispiel 39);
N-((3RS)-1-*tert*-Butylcarbonylmethyl-2,3-dihydro-2-oxo-5-phenyl-1H-1,4-benzodiazepin-3-yl)-N'-(3-dimethylaminophenyl)harnstoff (Beispiel 41);
N-((3RS)-1-*tert*-Butylcarbonylmethyl-2,3-dihydro-2-oxo-5-phenyl-1H-1,4-benzodiazepin-3-yl)-N'-(3-methoxyphenyl)harnstoff (Beispiel 43);
N-((3RS)-1-*tert*-Butylcarbonylmethyl-2,3-dihydro-2-oxo-5-phenyl-1H-1,4-benzodiazepin-3-yl)-N'-(3-nitrophenyl)harnstoff (Beispiel 45);
N-((3RS)-1-*tert*-Butylcarbonylmethyl-2,3-dihydro-2-oxo-5-phenyl-1H-1,4-benzodiazepin-3-yl)-N'-(3-formylaminophenyl)harnstoff (Beispiel 47);
N-((3R)-1-((2R)-2-Hydroxy-3,3-dimethylbutyl)-2,3-dihydro-2-oxo-5-phenyl-1H-1,4-benzodiazepin-3-yl)-N'-(3-methylphenyl)harnstoff (Beispiel 48);
N-((3R)-1-((2S)-2-Hydroxy-3,3-dimethylbutyl)-2,3-dihydro-2-oxo-5-phenyl-1H-1,4-benzodiazepin-3-yl)-N'-(3-methylphenyl)harnstoff;
N-((3RS)-1-*tert*-Butylcarbonylmethyl-2,3-dihydro-2-oxo-5-(2-chlorphenyl)-1H-1,4-benzodiazepin-3-yl)-N'-(3-methylphenylharnstoff (Beispiel 54);
N-((3RS)-1-Isopropylcarbonylmethyl-2,3-dihydro-2-oxo-5-phenyl-1H-1,4-benzodiazepin-3-yl)-N'-(3-methylphenyl)harnstoff (Beispiel 55);
N-((3R)-1-*tert*-Butylcarbonylmethyl-2,3-dihydro-2-oxo-5-phenyl-1H-1,4-benzodiazepin-3-yl)-N'-(3-methylaminophenyl)harnstoff (Beispiel 56);
N-((3RS)-1-*tert*-Butylcarbonylmethyl-2,3-dihydro-2-oxo-5-(2-pyridyl)-1H-1,4-benzodiazepin-3-yl)-N'-(3-methylaminophenyl)harnstoff (Beispiel 57);
N-((3R)-1-*tert*-Butylcarbonylmethyl-2,3-dihydro-2-oxo-5-phenyl-1H-1,4-benzodiazepin-3-yl)-N'-(3-(N-ethyl-N-methylamino)phenyl)harnstoff (Beispiel 58);
N-((3R)-1-*tert*-Butylcarbonylmethyl-2,3-dihydro-2-oxo-5-phenyl-1H-1,4-benzodiazepin-3-yl)-N'-(3-diethylaminophenyl)harnstoff (Beispiel 59);
N-((3RS)-1-*tert*-Butylcarbonylmetyl-2,3-dihydro-2-oxo-5-phenyl-1H-1,4-benzodiazepin-3-yl)-N'-(3-dimethylaminomethylphenyl)harnstoff (Beispiel 60);
N-((3RS)-1-*tert*-Butylcarbonylmethyl-2,3-dihydro-2-oxo-5-(2-pyridyl)-1H-1,4-benzodiazepin-3-yl)-N'-(3-(N-ethyl-N-methylamino)phenyl)harnstoff (Beispiel 61);
N-((3RS)-1-*tert*-Butylcarbonylmethyl-2,3-dihydro-2-oxo-5-(2-pyridyl)-1H-1,4,-benzodiazepin-3-yl)-N'-(3-dimethylaminomethylphenyl)harnstoff (Beispiel 62);
N-((3RS)-1-*tert*-Butylcarbonylmethyl-2,3-dihydro-2-oxo-5-(2-pyridyl)-1H-1,4-benzodiazepin-3-yl-N'-(3-diethylaminophenylharnstoff (Beispiel 63);
N-((3R)-1-*tert*-Butylcarbonylmethyl-2,3-dihydro-2-oxo-5-phenyl-1H-1,4-benzodiazepin-3-yl)-N'-(3-dimethylaminophenyl)harnstoff (Beispiel 64);
N-((3RS)-1-*tert*-Butylcarbonylmethyl-2,3-dihydro-2-oxo-5-(4-methylphenyl)-1H-1,4-benzodiazepin-3-yl)-N'-(3-methylphenyl)harnstoff (Beispiel 65);
N-((3R)-1-*tert*-Butylcarbonylmethyl-2,3-dihydro-2-oxo-5-phenyl-1H-1,4-benzodiazepin-3-yl)-N'-(3-aminophenyl)harnstoff (Beispiel 66);
N-((3R)-1-*tert*-Butylcarbonylmethyl-2,3-dihydro-2-oxo-5-(2-pyridyl)-1H-1,4-benzodiazepin-3-yl)-N'-(3-methylphenyl)harnstoff;
N-((3R)-1-*tert*-Butylcarbonylmethyl-2,3-dihydro-2-oxo-5-(2-pyridyl)-1H-1,4-benzodiazepin-3-yl)-N'-(3-aminophenyl)harnstoff;
N-((3R)-1-*tert*-Butylcarbonylmethyl-2,3-dihydro-2-oxo-5-(2-pyridyl)-1H-1,4-benzodiazepin-3-yl)-N'-(3-methylaminophenyl)harnstoff;
N-((3R)-1-*tert*-Butylcarbonylmethyl-2,3-dihydro-2-oxo-5-(2-pyridyl)-1H-1,4-benzodiazepin-3-yl)-N'-(3-dimethylaminophenyl)harnstoff;
N-((3RS)-1-*tert*-Butylcarbonylmethyl-2,3-dihydro-2-oxo-5-phenyl-1H-1,4-benzodiazepin-3-yl)-N'-(3-aminophenyl)harnstoff;
N-((3RS)-1-*tert*-Butylcarbonylmethyl-2,3-dihydro-2-oxo-5-phenyl-1H-1,4-benzodiazepin-3-yl)-N'-(3-methylaminophenyl)harnstoff;
N-((3RS)-1-*tert*-Butylcarbonylmethyl-7-chlor-2,3-dihydro-2-oxo-5-phenyl-1H-1,4-benzodiazepin-3-yl)-N'-(3-methylphenyl)harnstoff (Beispiel 67);
N-((3RS)-1-*tert*-Butylcarbonylmethyl-7-chlor-2,3-dihydro-2-oxo-5-phenyl-1H-1,4-benzodiazepin-3-yl)-N'-(3-dimethylaminophenyl)harnstoff (Beispiel 68);
N-((3RS)-1-*tert*-Butylcarbonylmethyl)-7-chlor-2,3-dihydro-2-oxo-5-(2-chlorphenyl)-1H-1,4-benzodiazepin-3-yl)-N'-(3-methylphenyl)harnstoff (Beispiel 69);
N-((3RS)-1-*tert*-Butylcarbonylmethyl-2,3-dihydro-8-methyl-2-oxo-5-phenyl-1H-1,4-benzodiazepin-3-yl)-N'-(3-methylphenyl)harnstoff (Beispiel 70);
N-((3RS)-1-*tert*-Butylcarbonylmethyl-2,3-dihydro-2-oxo-5-phenyl-1H-1,4-benzodiazepin-3-yl)-N'-(3-(N-ethyl-N-methylaminophenyl)harnstoff;
N-((3RS)-1-*tert*-Butylcarbonylmethyl-2,3-dihydro-2-oxo-5-phenyl-1H-1,4-benzodiazepin-3-yl)-N'-(3-diethylaminophenyl)harnstoff.

10. Medizinische Zusammensetzung, die als wirksamen Bestandteil eine Verbindung nach einem der vorherigen Ansprüche enthält.

11. Medizinische Zusammensetzung nach Anspruch 10, die als Gastrin- oder CCK-B-Rezeptorantagonist wirkt.

12. Medizinische Zusammensetzung nach Anspruch 11, die ein Arzneimittel zur Behandlung von Krankheiten ist, die durch das Versagen einer durch Gastrin gesteuerten physiologischen Funktion induziert werden, wie Magen- und Zwölffingerdarmgeschwüre, Gastritis, Refluxösophagitis, Magen- und Kolonkrebs sowie das Zollinger-Ellison-Syndrom.

13. Medizinische Zusammensetzung nach Anspruch 11, die ein Arzneimittel zur Behandlung von Krankheiten ist, die durch das Versagen einer durch den zentralen CCK-B-Rezeptor gesteuerten physiologischen Funktion induziert werden (z.B. zur Reduzierung von Angst oder zur Appetitregulierung).

14. Verfahren zur Herstellung von Benzodiazepin der allgemeinen Formel I oder eines pharmazeutisch wirksamen Salzes davon (wobei R¹ -CH₂CO(CH₂)ₐR⁵ ist und a, R², R³, R⁵, W und X der Definition in Anspruch 1 entsprechen), umfassend die Reaktion von 3-Aminobenzodiazepin V mit organischem Isocyanat R²-NCO:

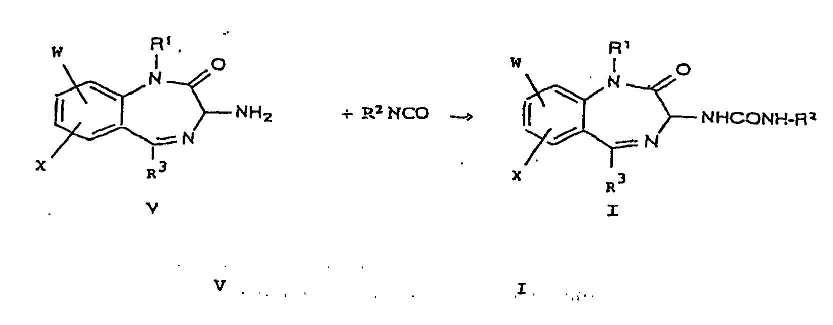

15. Verfahren zur Herstellung von Benzodiazepin der allgemeinen Formel I oder eines pharmazeutisch akzeptablen Salzes davon (wobei R¹ -CH₂CO(CH)₂ₐR⁵ ist und a, R², R³, R⁵, W und X der Definition in Anspruch 1 entsprechen), umfassend die Reaktion von 3-p-Nitrophenyloxycarbonylaminobenzodiazepin VI mit Amin R2-NH₂:

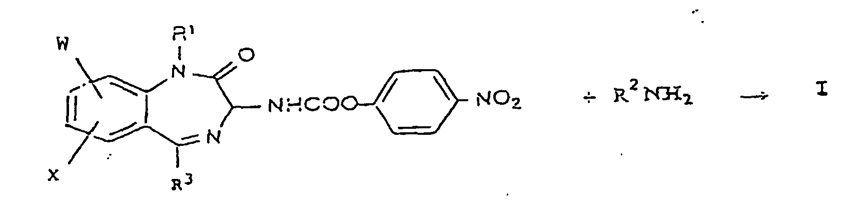

16. Verfahren zur Herstellung von Benzodiazepin der allgemeinen Formel I oder eines pharmazeutisch wirksamen Salzes davon (wobei R¹ -CH₂CHOH(CH₂)ₐR⁴ ist und a, R², R³, R⁴, W und X der Definition in Anspruch 1 entsprechen), umfassend die Reaktion von I mit einem Reduktionsmittel.

17. Verwendung einer Verbindung nach einem der Ansprüche 1 bis 9 zur Herstellung eines Medikaments zur Vorbeugung gegen oder Behandlung von eine(r) Krankheit, die durch das Versagen einer durch Gastrin oder den zentralen CCK-B-Rezeptor gesteuerten physiologischen Funktion induziert wird.

## Revendications

1. Dérivé de benzodiazépine de la formule I, ou un sel pharmaceutiquement acceptable de celui-ci : 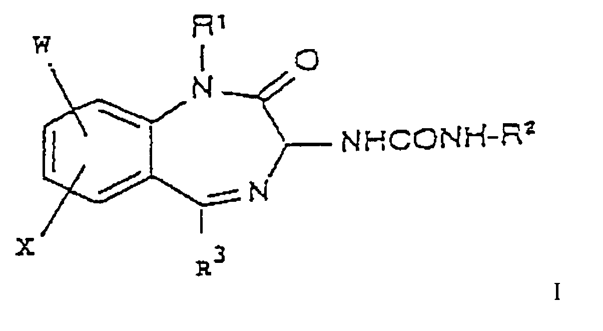 où :
(a) R¹ est -CH₂CHOH (CH₂)ₐR⁴ ou un groupe cétone -CH₂CO (CH₂)ₐR⁵ dans lequel a est 0 ou 1 et R⁴ et R⁵ sont sélectionnés parmi des groupes alkyle de jusqu'à 8 atomes de carbone;
(b) R² et R³ sont sélectionnés indépendamment parmi phényle et pyridyle optionnellement substitués, les substituants optionnels étant sélectionnés parmi des atomes d'halogène et des groupes hydroxy, amino, nitro, acide carboxylique, carboxamido et cyano et des groupes alkyle, alcoxy, alkylamino et dialkylamino dans lesquels le ou chaque groupe alkyle fait jusqu'à 8 atomes de carbone; et
(c) W et X sont sélectionnés indépendamment parmi des atomes d'halogène et d'hydrogène et des groupes alkyle C₁-C₈ et alcoxy C₁-C₈.

2. Composé selon la revendication 1, dans lequel R⁴ est un alkyle C₄-C₇ linéaire ou ramifié ou R⁵ est un alkyle C₁-C₃ ou tel que défini pour R⁴.

3. Composé selon la revendication 1 ou 2, dans lequel au moins l'un d'entre R² et R³ est phényle ou pyridyle non substitué ou substitué.

4. Composé selon la revendication 3, dans lequel ou moins l'un d'entre R² et R³ est phényle non substitué, monosubstitué ou disubstitué ou 2-, 3- ou 4-pyridyle non substitué, monosubstitué ou disubstitué.

5. Composé selon l'une quelconque des revendications précédentes, dans lequel
R¹ est -CH₂CO(CH₂)ₐR⁵,
R² est phényle non substitué; phényle ayant un substituant en méta choisi parmi F, Cl, Br, OH, OCH₃, NH₂, NMe₂, NO₂, Me, (CH₂)_{c}-CO₂H, CN, NHMe, NMeEt, NEt₂, CH₂NMe₂, NHCHO et (CH₂)_{c}-SO₃H, où c est 0-2; ou 2-, 3- ou 4-pyridyle optionnellement avec un substituant sélectionné parmi F, Cl, CH₃ et CO₂H; et
R³ est phényle ou 2-, 3- ou 4-pyridyle.

6. Composé selon l'une quelconque des revendications 1 à 4, dans lequel R¹ est - CH₂CHOH(CH₂)ₐR⁴ et R² et R³ sont tels que définis dans la revendication 5.

7. Composé selon l'une quelconque des revendications précédentes, dans lequel la configuration absolue à la position 3 du cycle benzodiazépine est R (comme montré dans IV).

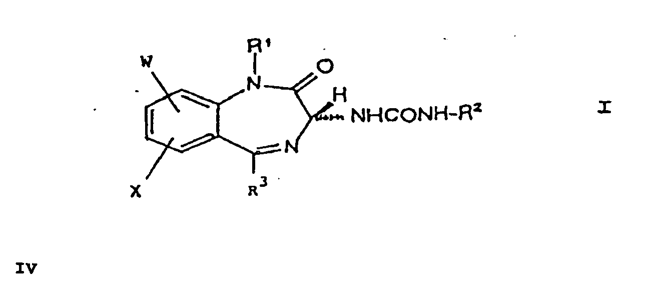

8. Composé selon l'une quelconque des revendications précédentes, dans lequel W et X sont H.

9. Au moins un composé sélectionné parmi les composés suivants selon la revendication 1, et des sels pharmaceutiquement acceptables de ceux-ci :
N-((3RS)-1-*tert*-butylcarbonylméthyl-2,3-dihydro-2-oxo-5-phényl-1H-1,4-benzodiazépin-3-yl)-N'-(3-méthylphényl) urée (Exemple 2);
N-((3RS)-1-diéthylméthylcarbonylméthyl-2,3-dihydro-2-oxo-5-phényl-1H-1,4-benzodiazépin-3-yl)-N'-(3-méthylphényl) urée (Exemple 3);
N-((3RS)-1-*tert*-butylcarbonylméthyl-2,3-dihydro-2-oxo-5-phényl-1H-1,4-benzodiazépin-3-yl)-N'-(3-cyanophényl) urée;
N-((3R)-1-*tert*-butylcarbonylméthyl-2,3-dihydro-2-oxo-5-phényl-1H-1,4-benzodiazépin-3-yl)-N'-(3-méthylphényl) urée (Exemple 26);
N-((3RS)-*tert*-butylcarbonylméthyl-2,3-dihydro-2-oxo-5-(2-pyridyl)-1H-1,4-benzodiazépin-3-yl)-N'-(3-méthylphényl) urée (Exemple 27);
N-((3RS)-1-*tert*-butylcarbonylméthyl-2,3-dihydro-2-oxo-5-phényl-1H-1,4-benzodiazépin-3-yl)-N'-(3-carboxyphényl) urée (Exemple 28);
N-((3RS)-1-*tert*-amylcarbonylméthyl-2,3-dihydro-2-oxo-5-phényl-1H-1,4-benzodiazépin-3-yl)-N'-(3-méthylphényl) urée (Exemple 36);
N-((3RS)-1-*tert*-amylcarbonylméthyl-2,3-dihydro-2-oxo-5-phényl-1H-1,4-benzodiazépin-3-yl)-N'-(3-carboxyphényl) urée (Exemple 37);
N-((3RS)-1-*tert*-butylcarbonylméthyl-2,3-dihydro-2-oxo-5-(2-pyridyl)-1H-1,4-benzodiazépin-3-yl)-N'-(3-diméthylaminophényl) urée (Exemple 38);
N-((3RS)-1-*tert*-butylcarbonylméthyl-2,3-dihydro-2-oxo-5-(2-pyridyl)-1H-1,4-benzodiazépin-3-yl)-N'-(3-carboxyphényl) urée (Exemple 39);
N-((3RS)-1-*tert*-butylcarbonylméthyl-2,3-dihydro-2-oxo-5-phényl-1H-1,4-benzodiazépin-3-yl)-N'-(3-diméthylaminophényl) urée (Exemple 41);
N-((3RS)-1-*tert*-butylcarbonylméthyl-2,3-dihydro-2-oxo-5-phényl-1H-1,4-benzodiazépin-3-yl)-N'-(3-méthoxyphényl) urée (Exemple 43);
N-((3RS)-1-*tert*-butylcarbonylméthyl-2,3-dihydro-2-oxo-5-phényl-1H-1,4-benzodiazépin-3-yl)-N'-(3-nitrophényl) urée (Exemple 45);
N-((3RS)-1-*tert*-butylcarbonylméthyl-2,3-dihydro-2-oxo-5-phényl-1H-1,4-benzodiazépin-3-yl)-N'-(3-formylaminophényl) urée (Exemple 47);
N-((3R)-1-((2R)-2-hydroxy-3,3-diméthylbutyl)-2,3-dihydro-2-oxo-5-phényl-1H-1,4-benzodiazépin-3-yl)-N'-(3-méthylphényl) urée (Exemple 48);
N-((3R)-1-((2S)-2-hydroxy-3,3-diméthylbutyl)-2,3-dihydro-2-oxo-5-phényl-1H-1,4-benzodiazépin-3-yl)-N'-(3-méthylphényl) urée;
N-((3RS)-1-*tert*-butylcarbonylméthyl-2,3-dihydro-2-oxo-5-(2-chlorophényl)-1H-1,4-benzodiazépin-3-yl)-N'-(3-méthylphényl) urée (Exemple 54);
N-((3RS)-1-isopropylcarbonylméthyl-2,3-dihydro-2-oxo-5-phényl-1H-1,4-benzodiazépin-3-yl)-N'-(3-méthylphényl) urée (Exemple 55);
N-((3R)-1-*tert*-butylcarbonylméthyl-2,3-dihydro-2-oxo-5-phényl-1H-1,4-benzodiazépin-3-yl)-N'-(6-méthylaminophényl) urée (Exemple 56);
N-((3RS)-1-*tert*-butylcarbonylméthyl-2,3-dihydro-2-oxo-5-(2-pyridyl)-1H-1,4-benzodiazépin-3-yl)-N'-(3-méthylaminophényl) urée (Exemple 57);
N-((3R)-1-*tert*-butylcarbonylméthyl-2,3-dihydro-2-oxo-5-phényl-1H-1,4-benzodiazépin-3-yl)-N'-(3-(N-éthyl-N-méthylamino)phényl) urée (Exemple 58);
N-((3R)-1-*tert*-butylcarbonylméthyl-2,3-dihydro-2-oxo-5-phényl-1H-1,4-benzodiazépin-3-yl)-N'-(3-diéthylaminophényle) urée (Exemple 59);
N-((3RS)-1-*tert*-butylcarbonylméthyl-2,3-dihydro-2-oxo-5-phényl-1H-1,4-benzodiazépin-3-yl)-N'-(3-diméthylaminométhylphényl) urée (Exemple 60);
N-((3RS)-1-*tert*-butylcarbonylméthyl-2,3-dihydro-2-oxo-5-(2-pyridyl)-1H-1,4-benzodiazépin-3-yl)-N'-(3-(N-éthyl-N-méthylamino)phényl) urée (Exemple 61);
N-((3RS)-1-*tert*-butylcarbonylméthyl-2,3-dihydro-2-oxo-5-(2-pyridyl)-1H-1,4-benzodiazépin-3-yl)-N'-(3-diméthylaminométhylphényl) urée (Exemple 62);
N-((3RS)-1-*tert*-butylcarbonylméthyl-2,3-dihydro-2-oxo-5-(2-pyridyl)-1H-1,4-benzodiazépin-3-yl)-N'-(3-diéthylaminophényl) urée (Exemple 63);
N-((3R)-1-*tert*-butylcarbonylméthyl-2,3-dihydro-2-oxo-5-phényl-1H-1,4-benzodiazépin-3-yl)-N'-(3-diméthylaminophényl) urée (Exemple 64);
N-((3RS)-1-*tert*-butylcarbonylméthyl-2,3-dihydro-2-oxo-5-(4-méthylphényl)-1H-1,4-benzodiazépin-3-yl)-N'-(3-méthylphényl) urée (Exemple 65);
N-((3R)-1-*tert*-butylcarbonylméthyl-2,3-dihydro-2-oxo-5-phényl-1H-1,4-benzodiazépin-3-yl)-N'-(3-aminophényl) urée (Exemple 66);
N-((3 R)-1-*tert*-butylcarbonylméthyl-2,3-dihydro-2-oxo-5-(2-pyridyl)-1H-1,4-benzodiazépin-3-yl)-N'-(3-méthylphényl) urée;
N-((3R)-1-*tert*-butylcarbonylméthyl-2,3-dihydro-2-oxo-5-(2-pyridyl)1-1H-1,4-benzodiazépin-3-yl)-N'-(3-aminophényl) urée;
N-((3R)-1-*tert*-butylcarbonylméthyl-2,3-dihydro-2-oxo-5-(2-pyridyl)-1H-1,4-benzodiazépin-3-yl)-N'-(3-méthylaminophényl) urée;
N-((3R)-1-*tert*-butylcarbonylméthyl-2,3-dihydro-2-oxo-5-(2-pyridyl)-1H-1,4-benzodiazépin-3-yl)-N'-(3-diméthylaminophényl) urée;
N-((3RS)-1-*tert*-butylcarbonylméthyl-2,3-dihydro-2-oxo-5-phényl-1H-1,4-benzodiazépin-3-yl)-N'-(3-aminophényl) urée;
N-((3RS)-1-*tert*-butylcarbonylméthyl-2,3-dihydro-2-oxo-5-phényl-1H-1,4-benzodiazépin-3-yl)-N'-(3-méthylaminophényl) urée;
N-((3RS)-1-*tert*-butylcarbonylméthyl-7-chloro-2,3-dihydro-2-oxo-5-phényl-1H-1,4-benzodiazépin-3-yl)-N'-(3-méthylphényl) urée (Exemple 67);
N-((3 RS)-1-*tert*-butylcarbonylméthyl-7-chloro-2,3-dihydro-2-oxo-5-phényl-1H-1,4-benzodiazépin-3-yl)-N'-(3-diméthylaminophényl) urée (Exemple 68);
N-((3RS)-1-*tert*-butylcarbonylméthyl-7-chloro-2,3-dihydro-2-oxo-5-(2-chlorophényl)-1H-1,4-benzodiazépin-3-yl)-N'-(3-méthylphényl) urée (Exemple 69);
N-((3RS)-1-*tert*-butylcarbonylméthyl-2,3-dihydro-8-méthyl-2-oxo-5-phényl-1H-1,4-benzodiazépin-3-yl)-N'-(3-méthylphényl) urée (Exemple 70);
N-((3RS)-1-*tert*-butylcarbonylméthyl-2,3-dihydro-2-oxo-5-phényl-1H-1,4-benzodiazépin-3-yl)-N'-(3-(N-éthyl-N-méthylaminophényl) urée;
N-((3 RS)-1-*tert*-butylcarbonylméthyl-2,3-dihydro-2-oxo-5-phényl-1H-1,4-benzodiazépin-3-yl)-N'-(3-diéthylaminophényl) urée.

10. Composition médicamenteuse contenant un composé de l'une quelconque des revendications précédentes en tant que principe actif.

11. Composition médicamenteuse selon la revendication 10, laquelle agit comme un antagoniste de la gastrine ou des récepteurs CCK-B.

12. Composition médicamenteuse selon la revendication 11, laquelle est un médicament pour le traitement de maladies induites par la défaillance d'une fonction physiologique contrôlée par la gastrine, telles que l'ulcère gastrique et duodénal, la gastrite, l'oesophagite par reflux, le cancer gastrique et du côlon et le syndrome de Zollinger-Ellison.

13. Composition médicamenteuse selon la revendication 11, laquelle est un médicament pour le traitement de maladies induites par la défaillance d'une fonction physiologique contrôlée par le récepteur CCK-B central (par exemple pour la réduction de l'anxiété ou pour la régulation de l'appétit).

14. Procédé de production de benzodiazépine de la formule générale I ou d'un sel pharmaceutiquement actif de celle-ci (où R¹ est -CH₂CO(CH₂)ₐR⁵ et a, R², R³, R⁵, W et X sont tels que définis dans la revendication 1), lequel comprend la réaction de 3-aminobenzodiazépine V avec un isocyanate organique R²-NCO :

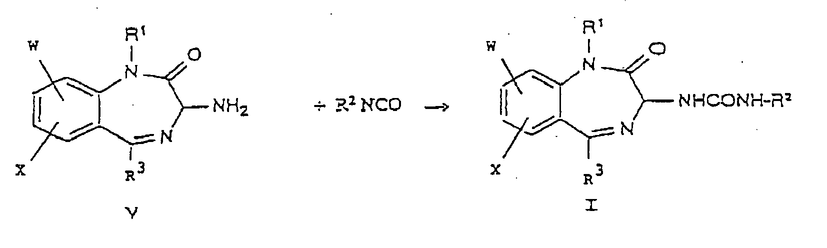

15. Procédé de production de benzodiazépine de la formule générale I ou d'un sel pharmaceutiquement acceptable de celle-ci (où R¹ est -CH₂CO(CH)₂ₐR⁵ et a, R², R³, R⁵, W et X sont tels que définis dans la revendication 1), lequel comprend la réaction de 3-p-nitrophényloxycarbonylamino benzodiazépine VI avec une amine R₂-NH₂ :

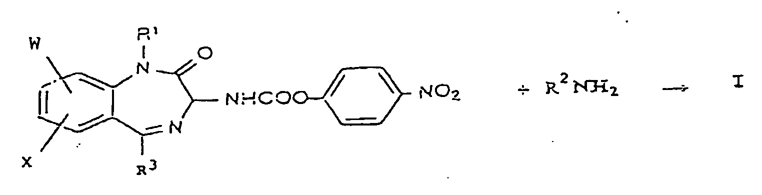

16. Procédé de production de benzodiazépine de la formule générale I ou d'un sel pharmaceutiquement actif de celle-ci (où R¹ est -CH₂CHOH(CH₂)ₐR⁴ et a, R², R³, R⁴, W et X sont tels que définis dans la revendication 1), lequel comprend la réaction de I avec un agent réducteur.

17. L'utilisation d'un composé selon l'une quelconque des revendications 1 à 9, dans la préparation d'un médicament pour la prévention ou le traitement d'une maladie induite par la défaillance d'une fonction physiologique contrôlée par la gastrine ou le récepteur CCK-B central.
